# EUROPEAN PATENT SPECIFICATION

(11) **EP 3 022 226 B1**
(45) Date of publication and mention of the grant of the patent: **05.12.2018**
(21) Application number: 14735574.7
(22) Date of filing: 04.07.2014
(51) Int. Cl.: C07K 16/28, C07K 14/545, C07K 16/32

(54) **TARGETED MODIFIED IL-1 FAMILY MEMBERS**
ANGEZIELTE MODIFIZIERTE MITGLIEDER DER IL-1-FAMILIE
MEMBRES DE LA FAMILLE IL-1 MODIFIÉS CIBLÉS

(30) Priority: 19.07.2013 EP 13306047
(43) Date of publication of application: 25.05.2016
(73) Proprietor: VIB VZW, 9052 Gent (BE); Universiteit Gent, 9000 Gent (BE); Centre National de la Recherche Scientifique, 75794 Paris Cedex 16 (FR); Université de Montpellier, 34090 Montpellier (FR); Centre Hospitalier Régional Universitaire de Montpellier, 34295 Montpellier (FR)
(72) Inventor: TAVERNIER, Jan, B-9860 Balegem (BE); GERLO, Sarah, B-9000 Gent (BE); PEELMAN, Frank, B-9050 Gentbrugge (BE); UZÉ, Gilles, F-34090 Montpellier (FR)
(74) Representative: Grund, Martin
(86) International application number: PCT/EP2014/064283
(87) International publication number: WO 2015/007542

(56) References cited:
- WO-A1-2009/003145
- WO-A1-2011/029870
- WO-A2-2010/036918
- WO-A2-2011/020783
- US-A- 5 914 254
- ROVERO S ET AL: "Insertion of the DNA for the 163 171 peptide of IL1b enables a DNA vaccine encoding p185neu to inhibit mammary carcinogenesis in Her-2/neu transgenic BALB/c mice", GENE THERAPY, NATURE PUBLISHING GROUP, GB, vol. 8, no. 6, 1 March 2001 (2001-03-01), pages 447-452, XP007916523, ISSN: 0969-7128
- DEFFAR KHALISSA ET AL: "Nanobodies - the new concept in antibody engineering", AFRICAN JOURNAL OF BIOTECHNOLOGY, ACADEMIC JOURNALS, NAIROBI, KENYA, vol. 8, no. 12, 17 June 2009 (2009-06-17), pages 2645-2652, XP009136924, ISSN: 1684-5315
- JANUSZ WESOLOWSKI ET AL: "Single domain antibodies: promising experimental and therapeutic tools in infection and immunity", MEDICAL MICROBIOLOGY AND IMMUNOLOGY, SPRINGER, BERLIN, DE, vol. 198, no. 3, 16 June 2009 (2009-06-16) , pages 157-174, XP019740594, ISSN: 1432-1831, DOI: 10.1007/S00430-009-0116-7
- NUTTALL S D ET AL: "Isolation and characterization of an IgNAR variable domain specific for the human mitochondrial translocase receptor Tom70", EUROPEAN JOURNAL OF BIOCHEMISTRY, WILEY-BLACKWELL PUBLISHING LTD, GB, vol. 270, no. 17, 1 September 2003 (2003-09-01), pages 3543-3554, XP003013540, ISSN: 0014-2956, DOI: 10.1046/J.1432-1033.2003.03737.X
- NUTTALL STEWART D ED - DIRK SAERENS ET AL: "CHAPTER 3: Overview and discovery of IgNARs and generation of VNARs", 1 January 2012 (2012-01-01), SINGLE DOMAIN ANTIBODIES. METHODS AND PROTOCOLS (BOOK SERIES: METHODS IN MOLECULAR BIOL, HUMANA PRESS, PAGE(S) 27 - 36, XP009170806, ISBN: 978-1-61779-967-9
- KOVALEVA MARINA ET AL: "Shark variable new antigen receptor biologics - a novel technology platform for therapeutic drug development", EXPERT OPINION ON BIOLOGICAL THE, INFORMA HEALTHCARE, UK, vol. 14, no. 10, 1 October 2014 (2014-10-01), pages 1527-1539, XP009187644, ISSN: 1744-7682, DOI: 10.1517/14712598.2014.937701
- SIMMONS D P ET AL: "Dimerisation strategies for shark IgNAR single domain antibody fragments", JOURNAL OF IMMUNOLOGICAL METHODS, ELSEVIER SCIENCE PUBLISHERS B.V.,AMSTERDAM, NL, vol. 315, no. 1-2, 31 August 2006 (2006-08-31), pages 171-184, XP028017599, ISSN: 0022-1759, DOI: 10.1016/J.JIM.2006.07.019 [retrieved on 2006-08-31]
- VICTOR A. STRELTSOV ET AL: "Structure of a shark IgNAR antibody variable domain and modeling of an early-developmental isotype", PROTEIN SCIENCE, vol. 14, no. 11, 1 November 2005 (2005-11-01), pages 2901-2909, XP055033681, ISSN: 0961-8368, DOI: 10.1110/ps.051709505
- DATABASE MEDLINE [Online] US NATIONAL LIBRARY OF MEDICINE (NLM), BETHESDA, MD, US; August 2011 (2011-08), JUAREZ KARLA ET AL: "Monoclonal antibodies for the identification and purification of vNAR domains and IgNAR immunoglobulins from the horn shark Heterodontus francisci.", Database accession no. NLM21851231 & JUAREZ KARLA ET AL: "Monoclonal antibodies for the identification and purification of vNAR domains and IgNAR immunoglobulins from the horn shark Heterodontus francisci.", HYBRIDOMA (2005) AUG 2011, vol. 30, no. 4, August 2011 (2011-08), pages 323-329, ISSN: 1557-8348
- DATABASE MEDLINE [Online] US NATIONAL LIBRARY OF MEDICINE (NLM), BETHESDA, MD, US; January 2013 (2013-01), CAMACHO-VILLEGAS TANYA ET AL: "Human TNF cytokine neutralization with a vNAR from Heterodontus francisci shark: a potential therapeutic use.", Database accession no. NLM23221782 & CAMACHO-VILLEGAS TANYA ET AL: "Human TNF cytokine neutralization with a vNAR from Heterodontus francisci shark: a potential therapeutic use.", MABS, vol. 5, no. 1, January 2013 (2013-01), pages 80-85, ISSN: 1942-0870
- KOVALENKO O V ET AL: "Atypical Antigen Recognition Mode of a Shark Immunoglobulin New Antigen Receptor (IgNAR) Variable Domain Characterized by Humanization and Structural Analysis", JOURNAL OF BIOLOGICAL CHEMISTRY, AMERICAN SOCIETY FOR BIOCHEMISTRY AND MOLECULAR BIOLOGY, INC, BETHESDA, MD, USA, vol. 288, no. 24, 14 June 2013 (2013-06-14), pages 17408-17419, XP002700029, ISSN: 1083-351X, DOI: 10.1074/JBC.M112.435289 [retrieved on 2013-04-30]
- DATABASE EMBASE [Online] ELSEVIER SCIENCE PUBLISHERS, AMSTERDAM, NL; 25 January 2013 (2013-01-25), GRIFFITHS K ET AL: "Shark variable new antigen receptor (VNAR) single domain antibody fragments: Stability and diagnostic applications", Database accession no. EMB-2013720679 & GRIFFITHS K ET AL: "Shark variable new antigen receptor (VNAR) single domain antibody fragments: Stability and diagnostic applications", ANTIBODIES 20130125 MDPI AG CHE, vol. 2, no. 1, 25 January 2013 (2013-01-25), pages 66-81, ISSN: 2073-446
- KOPSIDAS GEORGE ET AL: "RNA mutagenesis yields highly diverse mRNA libraries for in vitro protein evolution", BMC BIOTECHNOLOGY, BIOMED CENTRAL LTD. LONDON, GB, vol. 7, no. 1, 11 April 2007 (2007-04-11), page 18, XP021023598, ISSN: 1472-6750, DOI: 10.1186/1472-6750-7-18
- DATABASE MEDLINE [Online] US NATIONAL LIBRARY OF MEDICINE (NLM), BETHESDA, MD, US; November 2012 (2012-11), MÜLLER MISCHA R ET AL: "Improving the pharmacokinetic properties of biologics by fusion to an anti-HSA shark VNAR domain.", Database accession no. NLM23676205 & MÜLLER MISCHA R ET AL: "Improving the pharmacokinetic properties of biologics by fusion to an anti-HSA shark VNAR domain.", MABS, vol. 4, no. 6, November 2012 (2012-11), pages 673-685, ISSN: 1942-0870
- RAFAEL BOJALIL ET AL: "Anti-tumor necrosis factor VNAR single domains reduce lethality and regulate underlying inflammatory response in a murine model of endotoxic shock", BMC IMMUNOLOGY, BIOMED CENTRAL, LONDON, GB, vol. 14, no. 1, 2 April 2013 (2013-04-02), page 17, XP021146429, ISSN: 1471-2172, DOI: 10.1186/1471-2172-14-17

## Description

The present invention relates to a modified IL-1β, with reduced activity via its cytokine receptor, wherein said IL-1β is specifically delivered to target cells. The IL-1β is a mutant with reduced affinity to the IL-1 receptor, wherein said mutant IL-1β is specifically delivered to target cells. The targeting is realized by fusion of the modified IL-1β to a targeting moiety, which is a VHH or VNAR. The invention relates further to such targeted modified IL-1β for use in treating diseases.

The Interleukin-1 (IL-1) family consists of 11 structurally related family members (IL-1α, IL-1-β, IL-1Ra, IL-18, IL-33 and IL-1F5 to IL-1F10), that are among the most potent immune system signaling molecules, acting through a group of closely related receptors. All IL-1 receptors have a similar mode of activation: upon binding of ligand to the primary receptor subunit (i.e. IL-1R1 for IL-1α and β, IL-18R for IL-18 and ST2 for IL-33), a second receptor subunit is recruited (i.e. IL-1RAP for IL-1α and β, IL-18RAP for IL-18 and IL-1RAP for IL-33) and signalling is initiated via juxtaposition of the receptor subunits' cytoplasmic Toll/IL-1 receptor (TIR) domains. The dimerized TIR domains provide a docking platform for the MYD88 adaptor protein, which via recruitment of other intermediates leads to activation of the pro-inflammatory nuclear factor-κB (NF-κB) and mitogen-activated protein kinase (MAPK) pathways. The IL-1 family members are primarily produced by innate immune cells and act on a variety of cell types during the immune response (for review see Sims and Smith, 2010).

T lymphocytes are one of the main IL-1 family target cells and the potentiating effects of in particular IL-1α and IL-1β on the expansion and differentiation of different T cell subsets, in particular CD8+ T cells (Ben-Sasson, 2011; Ben-Sasson, 2013) and Th17 cells (Sutton et al., 2006; Acosta-Rodriguez et al., 2007; Dunne et al., 2010; Shaw et al., 2012) have been firmly established. Th17 cells are characterized by the production of IL-17 and play an important role in auto-immune disease and chronic inflammation (reviewed in Wilke et al., 2011). Among T cell subsets, Th17 cells express the highest levels of the IL-1R and IL-1 plays an important role in Th17 priming.

IL-18 is best known as an IFNγ-inducing cytokine with a potent action on Th1 cells and natural killer (NK) cells, on (Okamura et al., 1995; Takeda et al. 1998). In addition, IL-18 enhances neutrophil function (Leung et al., 2001). Several reports demonstrate IL-18 anti-tumour action in animal models (Micallef et al., 1997; Loeffler et al., 2008; Wigginton et al., 2002; Zaki et al., 2010) and recombinant human IL-18 therapy recently entered clinical trials to evaluate its efficacy for treatment of advanced cancer (Robertson et al., 2008). As opposed to IL-18, IL-33 acts primarily on Th2 cells (Schmitz et al., 2005) and mast cells (Allakhverdi et al., 2007), and recently was shown to act on CD8 + T cells to drive antiviral responses (Bonilla et al., 2012).

The other IL-1 family members are less well characterized, but in summary different IL-1 family members have specificities for different T-cell subsets or other cell types and hence different therapeutic applications.

Besides having indirect anti-tumour activity, via activation of T and NK cells, IL-1 family members were shown to have direct cytostatic properties, which were most convincingly demonstrated on human melanoma cells (Morinaga et al., 1990; Usui et al., 1991; Rangnekar et al., 1992).

In view of the contribution of several IL-1 family members to inflammatory processes, clinical interest has been mainly oriented towards the development of IL-1-antagonizing strategies (Dinarello et al., 2012). Nevertheless, exploitation of controlled agonistic IL-1 activity could have applications in different physiological/pathological processes, where immunostimulatory effects would be desirable. One of the main concerns regarding the use of IL-1 in immunostimulatory therapies, is its severe toxicity when administered systemically. However, when IL-1 action could be confined to a selected cellular population, the toxicity issue might be resolved, which opens up therapeutic perspectives.

For instance, although there has been a lot of interest on blocking Th17 responses in view of their pathogenic role in auto-immune conditions such as multiple sclerosis, rheumatoid arthritis and inflammatory bowel disease (Wilke et al., 2011), normal Th17 function is indispensable for protective immunity against a range of pathogens, including Mycobacterium tuberculosis (Khader et al., 2007), Klebsiella pneumoniae (Ye et al., 2001) and Bordetella pertussis (Higgins et al., 2006). As IL-1β stimulates Th17 function, the idea has been raised to use IL-1β as a T-cell adjuvant to enhance the response to weak vaccines (Ben-Sasson et al., 2011). Other applications could be the targeting of IL-1β or IL-33 to the CD8+ T-cell population to enhance antiviral responses or targeting IL-18 to Th1 cells or NK cells to promote anti-tumor activity.

WO 2011/029870, WO 2010/036918, US 5,914,254, WO 2011/020783, WO 2009/003145, and Rovero et al. (Gene Therapy 2001, 8:447-452) disclose different fusion constructs of IL-1β.

Surprisingly we found that it is possible to design IL-1 family modifications that are defective in activating their receptor, but, when fused to a targeting moiety, regain their activity on selected cell types by a concentration effect at the cell surface. The IL-1 mutants have a reduced affinity for their cognate receptors, and hence are unable to efficiently bind and activate their receptors. However, by fusing them to a targeting moiety (such as a nanobody) the activity of the mutant IL-1 family member is restored on cells expressing the cell surface target, recognized by the targeting moiety. Because the activation is confined to the selected targeted cell types only, no major systemic toxicity occurs.

The invention is defined by the appended claims.

A first aspect of the invention is composition comprising a targeting construct, comprising a modified IL-1β, characterized by a reduced affinity for its cytokine receptor, and a targeting moiety. A modified IL-1β means that the IL-1β has been changed to alter the affinity to its receptor, with as final result that the modified IL-1β has a reduced affinity for the receptor and a consequent reduced biological activity, as compared to the endogenous wild type cytokine that binds normally to the receptor. A reduced affinity and a consequent reduced biological activity as used here means that the modified IL-1 family cytokine has a biological activity of less than 70% of the biological activity of the IL-1 family cytokine, even more preferably less than 60% of the biological activity of the IL-1 family cytokine, more preferably less than 50% of the biological activity of the IL-1 family cytokine, more preferably less than 40% of the biological activity of the IL-1 family cytokine, more preferably less than 30% of the biological activity of the IL-1 family cytokine, more preferably less than 20% of the biological activity of the IL-1 family cytokine, more preferably less than 10% of the biological activity of the IL-1 family cytokine, most preferably less than 1% of the biological activity of the IL-1 family cytokine as compared to the IL-1 family cytokine that normally binds to the receptor. Preferably, the modified IL-1 family cytokine is a mutant of the wild type IL-1 family cytokine and the activity is compared with the wild type IL-1 family cytokine. The affinity and/or the activity can be measured by any method known to the person skilled in the art.

A preferred embodiment of the invention is a targeting construct, comprising a mutant IL-1β characterized by reduced affinity for the Interleukin-1 receptor type I (IL-1RI) and/or the interleukin-1 receptor accessory protein (IL-1RAcP) receptor, and a targeting moiety. The affinity of the mutant IL-1β to the receptor, in comparison to the affinity of the wild type IL-1β to the receptor can be measured by Scatchard plot analysis and computer-fitting of binding data (e.g. Scatchard, 1949) or by reflectometric interference spectroscopy under flow through conditions, as described by Brecht et al. (1993). The activity of the mutant IL-1β is typically measured using a bioassay (for example by the induction of cell death) or by measuring signaling events downstream of the receptor. Such signaling events can be the modification or nuclear translocation of NF-κB, or the induction of a selected reporter gene. Preferably, said mutant IL-1β has a biological activity of less than 70% of the biological activity of the wild type IL-1β, even more preferably less than 60% of the biological activity of the wild type IL-1β, more preferably less than 50% of the biological activity of the wild IL-1β, more preferably less than 40% of the biological activity of the wild IL-1β, more preferably less than 30% of the biological activity of the wild IL-1β, more preferably less than 20% of the biological activity of the wild IL-1β, more preferably less than 10% of the biological activity of the wild type, most preferably less than 1% of the wild type of which it is deduced (i.e. the wild type IL-1β of which the coding sequence has been mutated to obtain the mutant IL-1β). Said mutant comprises a mutation selected from the group consisting of R120X, Q131G, L145G, H146X, Q148X, F162A, K208E (wherein X can be any change in amino acid, preferably a non-conservative change). Even more preferably said mutation is selected from the group consisting of R120A, R120G, H146A, H146G, H146E, H146N, H146R, Q148E, Q148G, and Q148L. Most preferably said mutation is selected from the group consisting of R120G, H146N, H146R, Q148E, and Q148G. (numbering base on the human IL-1β sequence, genbank accession number NP_000567, version NP-000567.1, GI: 10835145).

Preferably, said targeting moiety is targeting to a marker expressed on an IL-1β receptor expressing cell, preferably a cell expressing IL1-RI. In one preferred embodiment, said targeting moiety is directed to a tissue specific marker.

The modified IL-1β is linked to a targeting moiety. "Linked" as used here may be by a covalent binding, or it may be by an affinity binding. A "targeting moiety" as used here is a binding molecule that can direct the fusion protein towards a binding site on a cell that is expressing a receptor for the IL-1β, by specific interaction between the binding site and the binding molecule. Said binding molecule is a variable domain of camelid heavy chain antibodies (VHH) or a variable domain of new antigen receptors (VNAR). Preferably, said targeting moiety consists of a single polypeptide chain and is not post-translationally modified. Even more preferably, said targeting moiety is a nanobody.

In a non-limiting example, said targeting moiety may be a bispecific antibody, directed to a binding site on the target cell for one specificity, and to the targeted cytokine, or to a tag fused to said cytokine for the other specificity. In another non-limiting example, the targeting moiety may be chemically linked to the mutant Interleukin-1β, or it may be a recombinant fusion protein. Preferably, said targeting construct is a recombinant fusion protein. The targeting moiety may be fused directly to the mutant IL-1β, or it may be fused with the help of a linker fragment, preferably a GGS linker. The targeting moiety may be fused at the aminoterminal or at the carboxyterminal end of the mutated IL-1β; preferably said targeting moiety is fused at the carboxyterminal extremity of the mutated IL-1β molecule. The targeting construct may further comprise other domains such as, but not limited to a tag sequence, a signal sequence, another cytokine or an antibody.

Another aspect of the invention is a composition according to the invention for use as a medicament. One preferred embodiment is a composition according to the invention for use in stimulation of the immune response. Indeed, it is know that IL-1 treatment can induce antigen expression on B-cells (Killar et al., 1989); likewise, IL-18 treatment is augmenting cellular and humoral immunities (Kinoshita et al., 2011). In a similar way, it has been demonstrated that IL-1 acts on T-cells to enhance the magnitude of in vivo immune responses (Ben-Sasson et al., 2011; Ben Sasson et al., 2013). Therefore, one preferred aspect of the invention is the composition according to the invention for use as an adjuvant in vaccination. The targeting construct according to the invention is especially interesting in this respect, as the pro-inflammatory effect of normal wild type IL-1 makes the application of IL-1 as such impossible.

Still another aspect of the invention is a composition according to the invention for use in treatment of cancer. Indeed, Morinaga et al., 1990, Usui et al., 1991and Rangnekar et al., 1992 have shown that IL-1 family members do have direct cytostatic properties, which were most convincingly demonstrated on human melanoma cells.

### BRIEF DESCRIPTION OF THE FIGURES

**Figure 1****:** Schematic representation of the IL-1β-nanobody fusion proteins
**Figure 2****:** Concentration dependency of the induction of the NFκB activity by wild type and mutant Q148G IL-1 Her2 nanobody fusions (A) and other selected mutants (B), in mock transfected cells, or cells transfected with signaling deficient Her2.
**Figure 3****:** Effect of wild type and mutant (Q148G, L145A/L147A, F162A/Q164E) IL-1 Her2 nanobody fusions on nuclear translocation of endogenous NF-κB p65 in mock transfected cells, or cells transfected with signaling deficient Her2.
**Figure 4****:** Induction of the NFκB activity by wild type and 5 different IL-1 mutants, fused to an anti-murine leptin receptor nanobody, on cells expressing the murine leptin receptor (mLR) or not (no mLR).
**Figure 5****:** Concentration dependency of the induction of the NFκB activity by IL1 double mutants fused to the Her2 nanobody in mock transfected cells, or cells transfected with signaling deficient Her2.

### EXAMPLES.

Examples not falling within the scope of the claims are for illustrative purposes only.

### Materials and methods to the examples

### Cloning of IL-1-nanobody fusion proteins.

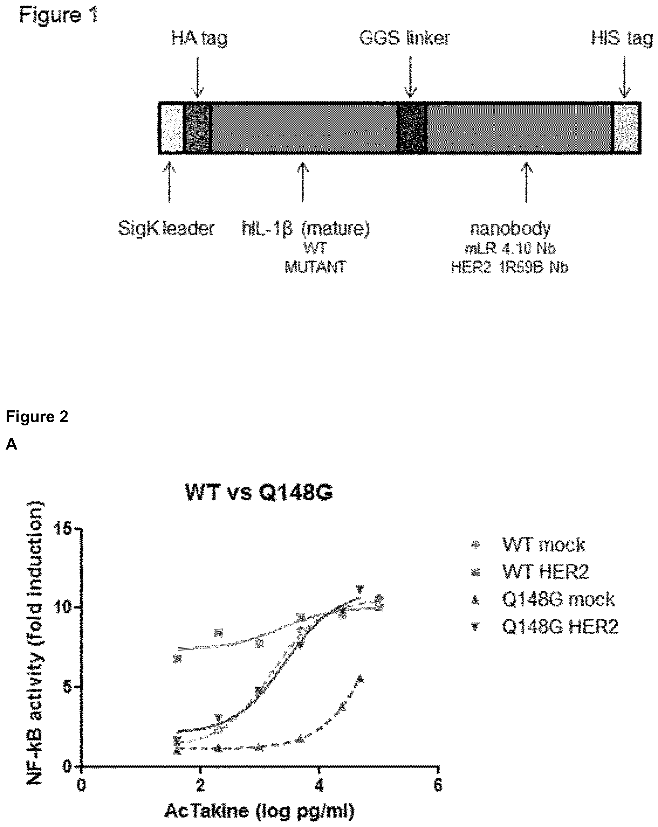

The 4-10 nanobody directed against the murine leptin receptor is described in Zabeau et al. (2012) and in the patent WO 2006/053883. The anti-Her2 nanobody 1R59B is described in Vaneycken et al. (2011). Both nanobodies were cloned with a C-terminal His tag in the pMET7 eukaryotic expression vector. A codon-optimized sequence encoding the mature IL-1β protein, preceded by the SigK leader peptide, and equipped with an N-terminal HA tag, was generated via gene synthesis (Invitrogen Gene Art). To generate the IL-1β-nanobody fusion proteins, the IL-1β sequence was cloned 5' to the nanobody sequence in pMet7, with a 13 x GGS linker separating the cytokine and nanobody moieties. (Fig. 1)

### IL-1β mutants.

IL-1β mutants expected to have reduced binding affinity for the IL-1R were selected based on literature and analysis of published crystal structures of human IL-1β complexed with its receptor. Mutations in the hIL-1β moiety were created via site-directed mutagenesis (QuickChange, Stratagene) using the mutagenesis primers as indicated in table I:

**Table I: mutants and primers used**

| | | **Fw primer** | **Rev primer** |
|---|---|---|---|
| **1** | **A117G/ P118G** | CCGACTACGCTGGCGGCAGTGACGGTGTCA GAAGCCTGAACTGC | GCAGTTCAGGCTTCTGACACCGTCACTG CCGCCAGCGTAGTCGG |
| **2** | **R120A** | CTGGCGGCAGCGCCCCTGTCGCTAGCCTGA ACTGCACCCTGCG | CGCAGGGTGCAGTTCAGGCTAGCGACA GGGGCGCTGCCGCCAG |
| **3** | **R120G** | GCGGCAGCGCCCCTGTCGGAAGCTTGAACT GCACCCTGC | GCAGGGTGCAGTTCAAGCTTCCGACAG GGGCGCTGCCGC |
| **4** | **L122A** | CGCTGGCGGCAGTGCCCCTGTCAGAAGCGC GAACTGCACCCTGCGGGACAGC | GCTGTCCCGCAGGGTGCAGTTCGCGCT TCTGACAGGGGCACTGCCGCCAGCG |
| **5** | **T125G/ L126G** | CGCCCCTGTCAGAAGCCTGAACTGCGGCGG CCGGGACAGCCAGCAGAAAAGC | GCTTTTCTGCTGGCTGTCCCGGCCGCC GCAGTTCAGGCTTCTGACAGGGGCG |
| **6** | **R127G** | AGAAGCCTGAACTGCACACTGGGGGACAGC CAGCAGAAAAGCCTGGTC | GACCAGGCTTTTCTGCTGGCTGTCCCCC AGTGTGCAGTTCAGGCTTCT |
| **7** | **Q130A** | CCCTGCGGGACAGCGCGCAGAAAAGCCTGG | CCAGGCTTTTCTGCGCGCTGTCCCGCA GGG |
| **8** | **Q130W** | CTGCACCCTGCGGGACAGCTGGCAGAAAAG CCTGGTCATGAGC | GCTCATGACCAGGCTTTTCTGCCAGCTG TCCCGCAGGGTGCAG |
| **9** | **Q131G** | CTGCGGGACAGCCAGGGGAAGAGCCTGGTC ATGAGCG | CGCTCATGACCAGGCTCTTCCCCTGGCT GTCCCGCAG |
| **10** | **K132A** | GCACCCTGCGGGACAGCCAGCAGGCTAGCC TGGTCATGAGCGGCC | GGCCGCTCATGACCAGGCTAGCCTGCT GGCTGTCCCGCAGGGTGC |
| **11** | **S137G/ Q138Y** | CAGCAGAAAAGCCTGGTCATGGGGTACCCCT ACGAGCTGAAGGCACTGC | GCAGTGCCTTCAGCTCGTAGGGGTACC CCATGACCAGGCTTTTCTGCTG |
| **12** | **L145G** | GCCCCTACGAGCTGAAGGCAGGTCATCTGCA GGGCCAGGACATGG | CCATGTCCTGGCCCTGCAGATGACCTG CCTTCAGCTCGTAGGGGC |
| **13** | **H146A** | CGAGCTGAAGGCACTGGCTCTTCAGGGCCA GGACATGG | CCATGTCCTGGCCCTGAAGAGCCAGTG CCTTCAGCTCG |
| **14** | **H146G** | CCTACGAGCTGAAGGCACTGGGTCTGCAGG | CCATGTCCTGGCCCTGCAGACCCAGTG |
| | | GCCAGGACATGG | CCTTCAGCTCGTAGG |
| **15** | **H146E** | GCTGAAGGCACTGGAGCTGCAGGGCCAGG | CCTGGCCCTGCAGCTCCAGTGCCTTCA GC |
| **16** | **H146N** | AGCTGAAGGCACTGAATCTGCAGGGCCAG | CTGGCCCTGCAGATTCAGTGCCTTCAGC T |
| **17** | **H146R** | CTGAAGGCACTGCGTCTGCAGGGCCAG | CTGGCCCTGCAGACGCAGTGCCTTCAG |
| **18** | **L145A/ L147A** | GCGGCCCCTACGAGCTGAAGGCAGCGCATG CGCAGGGCCAGGACATGG | CCATGTCCTGGCCCTGCGCATGCGCTG CCTTCAGCTCGTAGGGGCCGC |
| **19** | **Q148E** | GGCACTGCATCTGGAGGGCCAGGACAT | ATGTCCTGGCCCTCCAGATGCAGTGCC |
| **20** | **Q148G** | GAAGGCACTGCATCTGGGTGGCCAGGACAT GGAACAGC | GCTGTTCCATGTCCTGGCCACCCAGATG CAGTGCCTTC |
| **21** | **Q148L** | GCACTGCATCTGCTGGGCCAGGACATG | CATGTCCTGGCCCAGCAGATGCAGTGC |
| **22** | **Q148G/ Q150G** | CGAGCTGAAGGCACTGCATCTGGGGGCGG GGACATGGAACAGCAGG | CCTGCTGTTCCATGTCCCCGCCCCCCA GATGCAGTGCCTTCAGCTCG |
| **23** | **Q150G/ D151A** | GCACTGCATCTGCAGGGCGGGGCCATGGAA CAGCAGGTCGTGTTCAGC | GCTGAACACGACCTGCTGTTCCATGGCC CCGCCCTGCAGATGCAGTGC |
| **24** | **M152G** | GCACTGCATCTGCAGGGCCAGGACGGGAA CAGCAGGTGGTGTTCAGCATGAGC | GCTCATGCTGAACACCACCTGCTGTTCC CCGTCCTGGCCCTGCAGATGCAGTGC |
| **25** | **F162A** | CATGGAACAGCAGGTGGTGTTCAGCATGAGC GCCGTGCAGGGCGAGGAAAGCAACGAC | GTCGTTGCTTTCCTCGCCCTGCACGGC GCTCATGCTGAACACCACCTGCTGTTCC ATG |
| **26** | **F162A/ Q164E** | GCAGGTCGTGTTCAGCATGAGCGCCGTGGA GGGCGAGGAAAGCAATGACAAGATCC | GGATCTTGTCATTGCTTTCCTCGCCCTC CACGGCGCTCATGCTGAACACGACCTG C |
| **27** | **F166A** | CCGACTTCACCATGCAGGCCGTCTCCAGCGG CGGCAGCAGATCTGG | CCAGATCTGCTGCCGCCGCTGGAGACG GCCTGCATGGTGAAGTCGG |
| **28** | **Q164E/ E167K** | GCATGAGCTTCGTGGGGGCAAGGAAAGCA ATGACAAGATCCCCGTGGCC | GGCCACGGGGATCTTGTCATTGCTTTCC TTGCCCCCCACGAAGCTCATGC |
| **29** | **N169G/ D170G** | GCAGGGCGAGGAAAGCGGCGGCAAGATCCC CGTGGCCCTAGGCCTGAAAGAGAAG | CTTCTCTTTCAGGCCTAGGGCCACGGG GATCTTGCCGCCGCTTTCCTCGCCCTGC |
| **30** | **I172A** | GAAAGCAACGACAAGGCCCCCGTGGCCCTG GG | CCCAGGGCCACGGGGCCTTGTCGTTG CTTTC |
| **31** | **V174A** | GCAACGACAAGATCCCCGCGGCCCTGGGCC TGAAAG | CTTTCAGGCCCAGGGCCGCGGGATCT TGTCGTTGC |
| **32** | **K208E** | GCAGCTGGAAAGCGTGGATCCCAAGAACTAC CCCGAGAAAAAGATGGAAAAACGC | GCGTTTTTCCATCTTTTTCTCGGGGTAGT TCTTGGGATCCACGCTTTCCAGCTGC |
| **33** | **K209A** | CCCCAAGAACTACCCCAAGGCAAAGATGGAA AAGCGCTTCGTGTTCAAC | GTTGAACACGAAGCGCTTTTCCATCTTT GCCTTGGGGTAGTTCTTGGGG |
| **34** | **K209D** | GCAGCTGGAAAGCGTGGATCCCAAGAACTAC CCCAAGGACAAGATGGAAAAACGC | GCGTTTTTCCATCTTGTCCTTGGGGTAG TTCTTGGGATCCACGCTTTCCAGCTGC |
| **35** | **K209A/ K210A** | CCCCAAGAACTACCCCAAGGCAGCGATGGAA AAACGCTTCGTGTTC | GAACACGAAGCGTTTTTCCATCGCTGCC TTGGGGTAGTTCTTGGGG |
| **36** | **K219S** | AAAAACGCTTCGTGTTCAACAGCATCGAGAT CAACAACAAGCTC | GAGCTTGTTGTTGATCTCGATGCTGTTG AACACGAAGCGTTTTTT |
| **37** | **K219Q** | AAAAACGCTTCGTGTTCAACCAGATCGAGAT CAACAACAAG | CTTGTTGTTGATCTCGATCTGGTTGAAC ACGAAGCGTTTTT |
| **38** | **E221S** | GCTTCGTGTTCAACAAGATCTCGATCAACAAC AAGCTCGAGT | ACTCGAGCTTGTTGTTGATCGAGATCTT GTTGAACACGAAGC |
| **39** | **E221K** | CTTCGTGTTCAACAAGATCAAGATCAACAACA AGCTCGA | TCGAGCTTGTTGTTGATCTTGATCTTGTT GAACACGAAG |
| **40** | **K219S/ E221S** | GGAAAAACGCTTCGTCTTCAACAGCATCTCG ATCAACAACAAGCTCGAGTTCG | CGAACTCGAGCTTGTTGTTGATCGAGAT GCTGTTGAAGACGAAGCGTTTTTCC |
| **41** | **E221S/ N224A** | CGCTTCGTGTTCAACAAGATCTCGATCAACG CCAAGCTCGAGTTCGAG | CTCGAACTCGAGCTTGGCGTTGATCGAG ATCTTGTTGAACACGAAGCG |
| **42** | **N224S/ K225S** | CAACAAGATCGAGATCAACAGCAGCCTCGAA TTCGAGAGCGCCCAG | CTGGGCGCTCTCGAATTCGAGGCTGCT GTTGATCTCGATCTTGTTG |
| **43** | **E244K** | CCCCAACTGGTACATCAGTACTAGTCAGGCC AAGAATATGCCCGTGTTCC | GGAACACGGGCATATTCTTGGCCTGACT AGTACTGATGTACCAGTTGGGG |
| **44** | **N245Q** | CAGCACTAGTCAGGCCGAGCAGATGCCCGT CTTCCTGGGCGGCACC | GGTGCCGCCCAGGAAGACGGGCATCTG CTCGGCCTGACTAGTGCTG |
| **45** | **E244K/ N245Q** | CATCAGCACTAGTCAGGCCAAGCAGATGCCC GTCTTCCTGGGCGGCACC | GGTGCCGCCCAGGAAGACGGGCATCTG CTTGGCCTGACTAGTGCTGATG |
| **46 *** | **R120G/ Q131G** | GCGGCAGCGCCCCTGTCGGAAGCTTGAACT GCACCCTGC | GCAGGGTGCAGTTCAAGCTTCCGACAG GGGCGCTGCCGC |
| **47 *** | **R120G/** H146A | CGAGCTGAAGGCACTGGCTCTTCAGGGCCA GGACATGG | CCATGTCCTGGCCCTGAAGAGCCAGTG CCTTCAGCTCG |
| **49 *** | **R120G/ L145A/ L147A** | GCGGCCCCTACGAGCTGAAGGCAGCGCATG CGCAGGGCCAGGACATGG | CCATGTCCTGGCCCTGCGCATGCGCTG CCTTCAGCTCGTAGGGGCCGC |
| **48 *** | **R120G/ Q148G** | GCGGCAGCGCCCCTGTCGGAAGCTTGAACT GCACCCTGC | GCAGGGTGCAGTTCAAGCTTCCGACAG GGGCGCTGCCGC |
| **50** * | **R120G/ F162A/ Q164E** | GCAGGTCGTGTTCAGCATGAGCGCCGTGGA GGGCGAGGAAAGCAATGACAAGATCC | GGATCTTGTCATTGCTTTCCTCGCCCTC CACGGCGCTCATGCTGAACACGACCTG C |
| **51** | **R120G/ K208E** | GCAGCTGGAAAGCGTGGATCCCAAGAACTAC CCCGAGAAAAAGATGGAAAAACGC | GCGTTTTTCCATCTTTTTCTCGGGGTAGT TCTTGGGATCCACGCTTTCCAGCTGC |
| **52 **** | **Q131G/ Q148G** | CTGCGGGACAGCCAGGGGAAGAGCCTGGTC ATGAGCG | CGCTCATGACCAGGCTCTTCCCCTGGCT GTCCCGCAG |
| **53**** | **Q148G/ F162A/ Q164E** | GCAGGTCGTGTTCAGCATGAGCGCCGTGGA GGGCGAGGAAAGCAATGACAAGATCC | GGATCTTGTCATTGCTTTCCTCGCCCTC CACGGCGCTCATGCTGAACACGACCTG C |
| **54 **** | **Q148G/ K208E** | GCAGCTGGAAAGCGTGGATCCCAAGAACTAC CCCGAGAAAAAGATGGAAAAACGC | GCGTTTTTCCATCTTTTTCTCGGGGTAGT TCTTGGGATCCACGCTTTCCAGCTGC |

| | | | |
|---|---|---|---|
| * double/triple-mutants were created using R120G as template. ** double/triple-mutants were created using Q148G as template. | | | |

### Production of IL-1β fusion proteins.

IL-1β fusion proteins were produced in HEK293T cells. For small-scale production, HEK293T cells were seeded in 6-well plates at 400000 cells/well in DMEM supplemented with 10% FCS. After 24 hours, culture medium was replaced by medium with reduced serum (DMEM/5%FCS) and cells were transfected using linear PEI. Briefly, PEI transfection mix was prepared by combining 1 µg expression vector with 5 µg PEI in 160 µl DMEM, incubated for 10 minutes at RT and added to the wells dropwise. After 24 hours, transfected cells were washed with DMEM and layered with 1.5 ml OptiMem/well for protein production. Conditioned media were recuperated after 48 hours, filtered through 0.45 µ filters and stored at -20°C. IL-1β content in the conditioned media was determined by Elisa according to the manufacturer's instructions (R&D Systems).

### NF-κB reporter gene assay.

To assess IL-1R activation, we used HEK-Blue™ IL-1β cells that stably express the IL-1R (Invivogen) and transfected them transiently with an NF-κB luciferase reportergene. Briefly, HEK-Blue™ IL-1β cells were seeded in culture medium (DMEM/10%FCS) in 96-well plates (10000 cells/well) and transfected the next day using the calciumphosphate precipitation method with the indicated amounts of expression plasmids and 5 ng/well of the 3κB-Luc reportergene plasmid (Vanden Berghe et al., 1998). 24 hours post-transfection, culture medium was replaced by starvation medium (DMEM) and 48 hours post-transfection, cells were induced for 6 hours with fusion proteins. After induction, cells were lysed and luciferase activity in lysates was determined using the Promega Firefly Luciferase Assay System on a Berthold centro LB960 luminometer.

### Analysis of NF-κB nuclear translocation via confocal microscopy.

For confocal imaging, 10⁵ HEK293-T cells/well (in 6-well plate) were seeded on glass coverslips (Zeiss), coated with poly-L-lysine (Sigma). The next day, cells were transfected with 200 ng/well of empty vector or HER2Δcyt expression plasmid using the calcium phosphate precipitation method. After 48 hours, cells were treated for 30 minutes with vehicle (medium) or IL1-Her2 nanobody fusion protein (10 ng/ml). Next, cells were rinsed with 1×PBS and fixed for 15 minutes at room temperature in 4% paraformaldehyde. After three washes with 1xPBS, cells were permeabilized with 0.1% Triton X-100 in 1xPBS for 10 minutes and blocked in 1% BSA in 1xPBS for another 10 minutes at room temperature. Samples were then incubated for 1 hour at 37°C with rabbit anti-p65 antibody (Santa Cruz C20, diluted 1:800) and mouse anti-Flag Antibody (Sigma M2, 1:2000). After four washes in 1x PBS, cells were incubated for 1 hour at room temperature with anti-rabbit Alexa 488 and anti-mouse Alexa 594 fluorochrome-conjugated secondary antibodies (both diluted 1:800). After secondary antibody incubation, cells were washed four times in 1xPBS and nuclei were stained with DAPI (2 µg/ml). After a final wash step in 1xPBS, coverslips were mounted using propyl gallate. Images were acquired using a 60x 1.35 NA objective on an Olympus IX-81 laser scanning confocal microscope and analyzed using Fluoview 1000 software.

### Example 1: IL-1β-ligand and IL-1β-nanobody fusion proteins.

Fig. 1 shows a scheme of the IL-1β-nanobody fusion proteins constructed with either WT hIL-1β or the hIL1β mutants described in table I.

### Example 2: IL-1β activity of selected mutant IL-1β-nanobody fusions is restored on cells expressing the Nb targets.

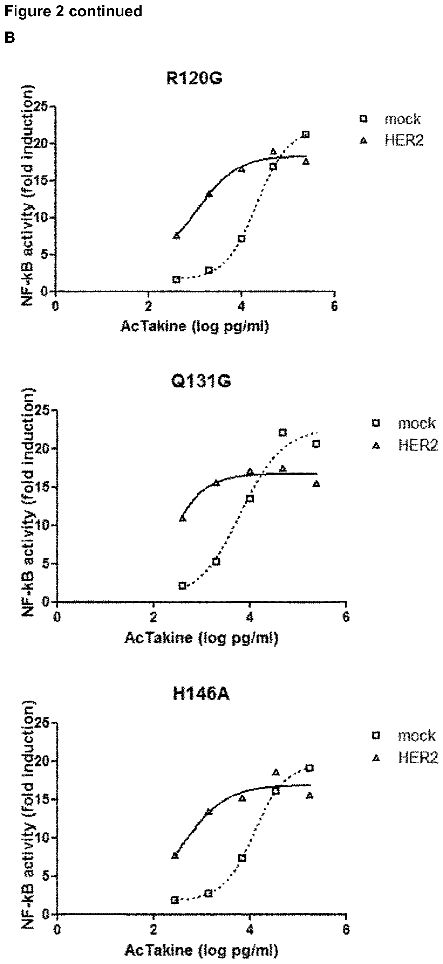

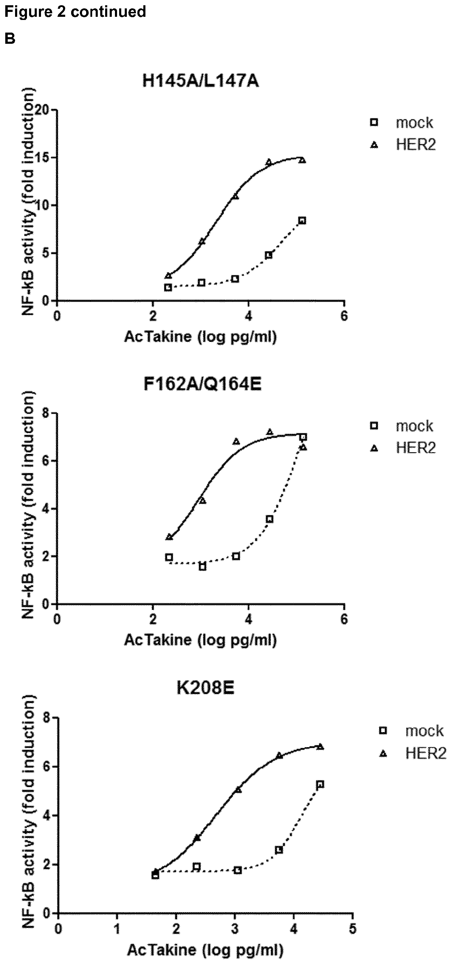

Wild type IL-1β and 45 IL-1β mutants (Table I) were fused to a well-characterized nanobody recognizing Her2 (1R59B). The IL-1β-nanobody fusion proteins were tested on HEK-Blue™ IL-1β cells, transiently transfected with an NF-κB reportergene plasmid (5 ng/well) and a Her2Δcyt (signalling-deficient) expression plasmid (2 ng/well). Cells were treated for 6 hours with IL-1β-Her2 nanobody fusions (dose response ranging from 0,4 to 250 ng/ml). As demonstrated in Fig. 2A, the IL-1β-Q148G-Her2 nanobody fusion displayed a reduced ability to activate NF-κB as compared to the WT IL1-β-Her2 nanobody fusion. Importantly, targeting of the Q148G mutant to Her2Δcyt-expressing cells restored its activity and produced a dose-response curve for NF-KB activation that perfectly parallels that of the WT IL-1β on mock-transfected cells. Also evident from this figure is a strong targeting effect for the WT IL-1β Her2 nanobody fusion. Similar "activation by targeting" effects were observed for six other IL-1β mutants (R120G, Q131G, H146A, H145A/L147A, F162A/Q164E and K208E) fused to the Her2 nanobody (Fig. 2B).

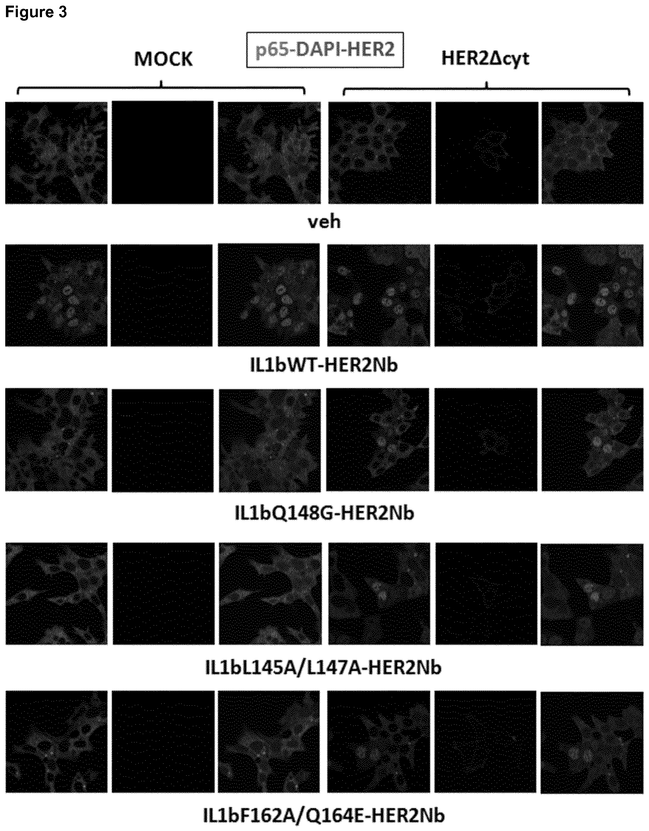

To obtain further proof for the "activation by targeting" concept, we next explored whether we could visualize the selective activation of NF-κB in Her2-expressing cells by the IL-1β-Her2 nanobody fusions via confocal microscopy. We measured activation of endogenous NF-κB by assaying its nuclear translocation. As evident from Fig. 3, only the WT IL-1β-Her2 nanobody fusion promoted translocation of endogenous NF-κB in cells that do not express Her2. Whereas they did not promote detectable NF-κB translocation in mock-transfected cells, the three tested mutant IL1-β-Her2 nanobody fusions triggered NF-κB nuclear translocation in cells that also stained positive for Her2, indicating they only act on targeted cells.

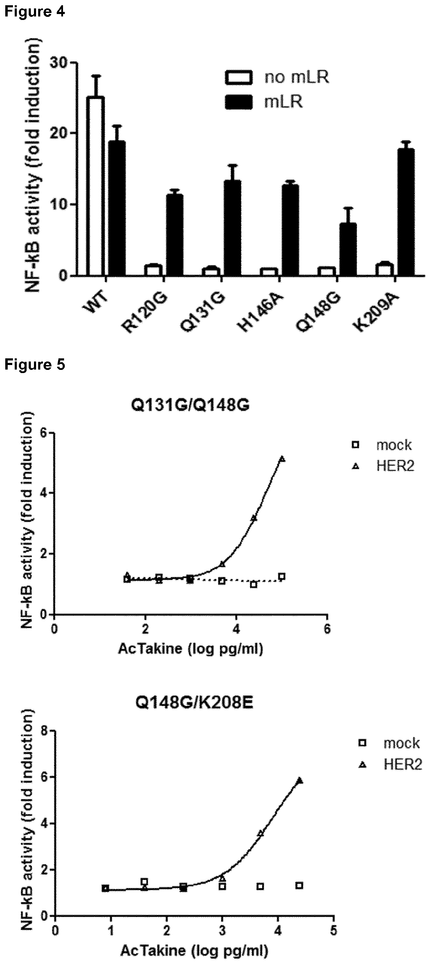

To evaluate whether the "activation by targeting" concept also works using a nanobody to an unrelated membrane protein, we fused WT IL-1β and five of the disabled IL-1β mutants (R120G, Q131G, H146A, Q148G, K209A) to a previously characterized nanobody recognizing the mLR (4-10). An experiment similar to that reported for the IL-1β-Her2 nanobody fusion (Fig. 2) was performed using HEK-Blue™ IL-1β cells, transiently transfected with a mLR expression plasmid (10 ng/well). Similar to the results obtained with the Her2 nanobody fusion proteins, all investigated mutant IL-1β nanobody fusions (tested at 12.5 ng/ml) showed a reduced ability, as compared to the WT fusion, to activate NF-κB on cells that do not express mLRs. However, targeting by the mLR nanobody moiety partially restored the activity of the selected mutants (Fig. 4).

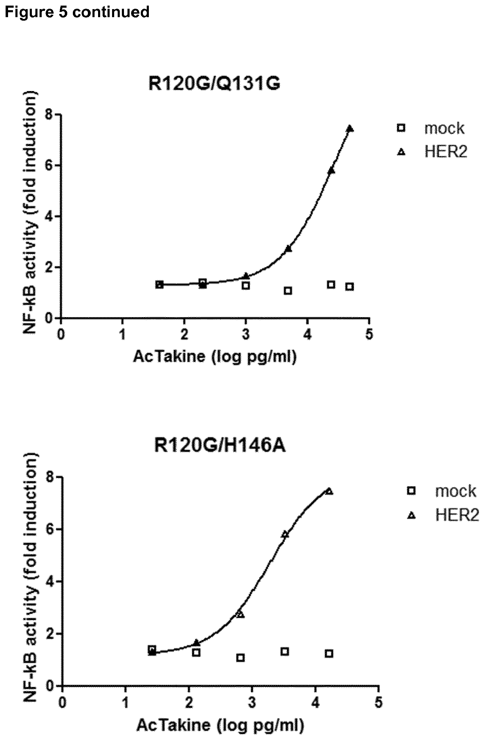

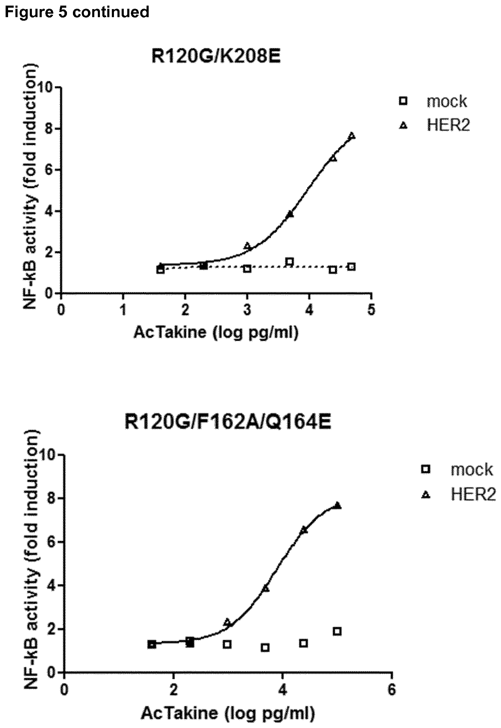

Because the IL-1β mutants described above retained significant residual biological activity, we combined different mutations to obtain double/triple mutants with reduced basal activity. Nine double/triple mutants were tested (cf. table I mutants 46 to 54) and from these, six mutant proteins (Q131G/Q148G, Q148G/K208E, R120G/Q131G, R120G/Q131G, R120G/H146A, R120G/K208E, R120G/F162A/Q164E) displayed no residual activity (using the same assay for measuring NF-κB as in Fig. 2) on Her2-negative cells, whilst partially restored activity was apparent on cells overexpressing Her2Δcyt (Fig. 5).

These data altogether indicate that targeting partially inactive mutant IL-1β, by fusing it to a nanobody recognizing a cell surface receptor, can restore its activity on nanobody target cells, probably by forced receptor interaction through a membrane concentration effect. The fact that activation by targeting can be accomplished using nanobodies recognizing different classes of membrane proteins indicates broad applicability of the "activation by targeting" concept.

Because these data provide proof of concept for the ability of targeting mutant IL-1 family members to selected cell types, restoring their activity on these target cells only, nanobodies are produced that allow targeting IL-1 family members to physiologically relevant IL-1β target cells. In view of the important role of IL-1 family members as T- and NK-cell activators, the nanobodies are designed to specifically target IL-1 to T- and NK-cell subsets. More specifically nanobodies targeting CCR6, which are predominantly expressed on Th17 cells as well as nanobodies targeting CD8 on cytotoxic T cells are developed and fused to the members of the IL1-family, preferably IL-1β.

### Example 3: Effect of lL-1β-nanobody fusions on IL-17 production by primary human T cells.

Primary human T cells were isolated from buffy coats. First, PBMC's were isolated by lymphoprep density gradient centrifugation and incubated O/N with 0.5 ng/ml rhlL-2 for recovery. Next, T-cells were isolated using the pan-T cell isolation kit (Miltenyi Biotec) according to the manufacturer's instructions. Briefly, T cells were resuspended (1 x 10⁶/ml) in RPMI-1640 supplemented with 10 %FCS and CD3/CD28 activating microbeads (Miltenyi Biotec). Next, cells (100 µl/well) were plated in U-bottom 96-well plates and stimulated for 96 hours with the indicated concentrations of IL-1β variants. After an additional 6 hours stimulation with PMA/ionomycin (both at 100 nM), supernatants were recovered and IL-17 levels were determined by Elisa (R&D Systems). Additional cytokines are evaluated via Luminex technology.

For selected mutant IL-1β-nanobody fusions (e.g. with a nanobody targeting CCR6) target cell-specific IL-17 and IFNγ production are evaluated by intracellular staining using a flow cytometric approach.

Also, to corroborate selectivity for the Th17 population, binding to PBMC subpopulations is measured via double staining using the Flag tag and selected CD markers, followed by flow cytometric analysis.

Finally, in a clinically relevant *in vitro* model of human Th17 cell function, the adjuvant activity of the IL-1β-nanobody fusions is assessed. In view of the need for more efficacious vaccines against Bordetella pertussis (or adjuvants for the existing vaccines), we determined whether the selected fusion proteins enhance the human Th17 response in a coculture model of naive T cells with B. pertussis-treated monocyte-derived dendritic cells (MDDCs). Human MDDCs are isolated from buffy coats (using the monocyte isolation kit II, Miltenyi Biotec), treated with different ratios of B. pertussis for 48 hours and then cocultured with naive allogeneic T cells for 12 days. After restimulation with anti-CD3/anti-CD28, the cytokine profiles in supernatants are determined using Elisa/Luminex technology (cfr. supra).

### Example 4: Effect of IL-1β-nanobody fusions on CTLs

To assess whether IL-1β-CD8 nanobody fusions can specifically enhance the function of CD8+ T cells, human PBMC's are isolated by lymphoprep density gradient centrifugation from buffy coats and stimulated for 24 hours with CD3/CD28 activating microbeads (Miltenyi Biotec) in combination with wt or mutant IL1β-CD8 Nb fusions. The effect of these fusion proteins on CD8+ T cell activation is evaluated by performing intracellular staining for active (phosphorylated) NF-κB and IFNγ. In addition, to investigate whether the IL-1β-nanobody fusions affect CTL degranulation, PBMC's (2 x10⁶cells/ml) are differentiated for 48 hours in the presence of phytohaemagglutinin (PHA, 1 µg/ml) and IL-2 (100 IU/ml) in combination with increasing doses of the IL-1β fusion proteins. Next, to induce degranulation, cells are stimulated for 3 hours with CD3/CD28 dynabeads and analysed by flow cytometry. Degranulation is measured via detection of cell surface CD107a, a well-established marker for natural killer activity. In all flow cytometric analyses on leukocyte pools, anti-CD8 staining is included to allow monitoring of the cell-type specificity of the IL-1β-CD8 Nb effects.

Finally to assess whether the IL-1β-CD8 nanobody fusions promote anti-tumor activity *in vivo,* C57BL/6 mice are injected subcutaneously with TC1 tumor cells, which produce the E6 and E7 antigenic oncoproteins from HPV16. This model was previously used to demonstrate that IL-1β promotes CD8+ T cell-mediated, antigen-specific, anti-tumor responses (Ben-Sasson, 2013). Briefly, mice are immunized four days after tumor injection with a vaccine containing the HPV16E7₄₉₋₅₇ peptide, combined with DOTAP and LPS, and with our without WT or mutant IL-1β-CD8 Nb fusions or IL-1β-GFP Nb fusions. Tumor size is monitored for 18 days post-immunization.

### Example 5: In vivo experiments - Vaccine adjuvans effect.

In a first series of experiments C57BL/6 mice are treated iv/ip with different doses of WT and mutant IL-1β-nanobody fusions and unfused IL-1β, to monitor acute toxicity. Venous blood is collected at different times post treatment by tail venopuncture and the cytokine profile in serum is determined by Luminex assay. In addition, via flow cytometric analysis intracellular cytokine levels (IL-17, IFNγ) and activation of IL-1R (as assessed by measuring phospho-NF-κB levels) are determined in selected leukocyte subsets.

When optimal doses have been established, their adjuvant activity is assessed in a murine vaccination protocol. Briefly, C57BL/6 mice are immunized ip with acellular pertussis vaccine (Pa). The Pa vaccine is composed of 5 µg/mouse of purified recombinant detoxified pertussis toxin (PT9K/129G) + filamentous hemagglutinin (FHA) (composition according to Brereton et al., 2011). 24 hours after immunization, selected mutant IL1β-Nb or PBS are administered ip or iv. Animals are boosted after 28 days. One set of animals is sacrificed 14 days after the second immunization and splenocytes are isolated and restimulated *in vitro* with medium or FHA for 3 days. Cytokine levels in culture supernatants (IL-17, IFNγ, IL-2, IL-10, IL-5, IL-4, etc.) are determined via Luminex technology. A second set of mice is challenged with B. pertussis on day 14 post-boost and sacrificed 2h and 5 and 10 days post-challenge. Lungs are isolated and CFU in lung homogenates will be quantified on Bordet-Gengou agar plates. Cytokine levels in lung homogenates are determined as in splenocyte supernatants.

In addition, blood is sampled (from the tail vene) before immunization and then every 14 days for determination of B. pertussis-specific IgG levels in serum.

### Example 6: Direct antitumor effect of IL-1β-nanobody fusions

To investigate the direct anti-tumour activity of selected IL1-nanobody fusions, we use human A375 melanoma cells, which were shown to be highly susceptible to IL-1-induced cytostatic effects (Morinaga et al., 1990). To allow targeting of mutant IL-1 family members to the A375 cells, a stable A375 clone expressing a cell surface marker to which high-affinity nanobodies are already available (i.e. CD20) is generated. The sensitivity of this cell line, as compared to the parental A375 cells, to the antiproliferative effect of the mutant IL1-nanobody fusion, is investigated in vitro using the XTT proliferation assay. In vivo anti-tumour activity of the mutant IL-1-nanobody fusions is investigated using an A375 xenotransplant model. Briefly, athymic nude mice are inoculated subcutaneously with A375 cells (parental or expressing a surface marker for targeting) and tumor growth is monitored for four weeks in animals treated with PBS or mutant IL1-nanobody fusions.

### Example 7: Extension of principle to IL18: application in tumor models

To assess the indirect anti-tumour activity of IL1 family members, experiments are conducted to address the efficacy of selected mutant IL-18-nanobody fusions using the Meth A syngeneic mouse sarcoma model according to the protocol that was used previously to demonstrate anti-tumour activity of IL-18 (Micallef et al., 1997). IL18 variants used in these experiments consist of mutant IL-18s fused to nanobodies targeting immune cells with tumoricidal properties (i.e. CTLs, NK-cells). The mice are treated with the construct, and a significant reduction of the tumor is noted when compared to the mock treated control.

### REFERENCES

Acosta-Rodriguez EV, Napolitani G, Lanzavecchia A, Sallusto F. (2007) Interleukins 1beta and 6 but not transforming growth factor-beta are essential for the differentiation of interleukin 17-producing human T helper cells. Nat Immunol. 8:942-9.
Allakhverdi Z, Smith DE, Comeau MR, Delespesse G. (2007) Cutting edge: The ST2 ligand IL-33 potently activates and drives maturation of human mast cells. J Immunol. 179:2051-4.
Ben-Sasson SZ, Caucheteux S, Crank M, Hu-Li J, Paul WE. (2011) IL-1 acts on T cells to enhance the magnitude of in vivo immune responses. Cytokine.56:122-5.
Ben-Sasson SZ, Hogg A, Hu-Li J, Wingfield P, Chen X, Crank M, Caucheteux S, Ratner-Hurevich M, Berzofsky JA, Nir-Paz R, Paul WE.(2013) IL-1 enhances expansion, effector function, tissue localization, and memory response of antigen-specific CD8 T cells. J Exp Med. 210:491-502.
Blake, A.W., McCartney, L., Flint, J., Bolam, D.N., Boraston, A.B., Gilbert, H.J. and Knox, J.P. (2006) Understanding the biological rationale for the diversity of cellulose-directed carbohydrate-binding molecules in prokaryotic enzymes. J. Biol. Chem. 281, 29321-29329.
Bonilla WV, Fröhlich A, Senn K, Kallert S, Fernandez M, Johnson S, Kreutzfeldt M, Hegazy AN, Schrick C, Fallon PG, Klemenz R, Nakae S, Adler H, Merkler D, Löhning M, Pinschewer DD.(2012). The alarmin interleukin-33 drives protective antiviral CD8+ T cell responses. Science. 335:984-9.
Brecht A., Gauglitz G., Polster J. (1993). Interferometric immunoassay in a FIA-system - A sensitive and rapid approach in label-free immunosensing., Biosens Bioelectron 8 : 387-392.
Brereton CF, Sutton CE, Ross PJ, Iwakura Y, Pizza M, Rappuoli R, Lavelle EC, Mills KH. (2011). Escherichia coli heat-labile enterotoxin promotes protective Th17 responses against infection by driving innate IL-1 and IL-23 production. J Immunol. 2011 May 15;186(10):5896-906.
Dimitrov, D.S. (2009) Engineered CH2 domains (nanoantibodies). mAbs 1, 26-28.
Dinarello CA, Simon A, van der Meer JW. (2012). Treating inflammation by blocking interleukin-1 in a broad spectrum of diseases. Nat Rev Drug Discov. 11:633-52.
Dunne A, Ross PJ, Pospisilova E, Masin J, Meaney A, Sutton CE, Iwakura Y, Tschopp J, Sebo P, Mills KH. (2010). Inflammasome activation by adenylate cyclase toxin directs Th17 responses and protection against Bordetella pertussis. J Immunol. 185:1711-9.
Higgins SC, Jarnicki AG, Lavelle EC, Mills KH. TLR4 mediates vaccine-induced protective cellular immunity to Bordetella pertussis: role of IL-17-producing T cells. J Immunol. 2006 Dec 1;177(11):7980-9.
Khader SA, Bell GK, Pearl JE, Fountain JJ, Rangel-Moreno J, Cilley GE, Shen F, Eaton SM, Gaffen SL, Swain SL, Locksley RM, Haynes L, Randall TD, Cooper AM. (2007). IL-23 and IL-17 in the establishment of protective pulmonary CD4+ T cell responses after vaccination and during Mycobacterium tuberculosis challenge. Nat Immunol. 2007 8:369-77.
Killar, L.M., Hatfield, C.A., Carding, S.R., Pan, M., Winterrowd, G.E. and Bottomly, K. (1989) In vivo administration of interleukin 1 elecits an increased Ia antigen expression on B cells throught the production of interleukin 4. Eur. J. Immunol. 19, 2205-2210.
Kinoshita, M., Miyazaki, H., Ono, S., Inatsu, A., Nakashima, H., Tsujimoto, H., Shinomiya, N., Saitoh, D. and Seki, S. (2011). Enhancement of neutrophil function by interleukin 18 therapy protects burn-injuredf mice from methicillin-resistant Staphylococcus aureus. Infect. Immun. 79, 2670-2680.
Kolmar, H. (2008) Alternative binding proteins: biological activity and therapeutic potential of cysteine-knot miniproteins. FEBS J. 275, 2684-2690.
Leung BP, Culshaw S, Gracie JA, Hunter D, Canetti CA, Campbell C, Cunha F, Liew FY, Mclnnes IB. (2001). A role for IL-18 in neutrophil activation. J Immunol. 167:2879-86.
Loeffler M, Le'Negrate G, Krajewska M, Reed JC. (2008). IL-18-producing Salmonella inhibit tumor growth. Cancer Gene Ther. 15:787-94.
Micallef MJ, Tanimoto T, Kohno K, Ikeda M, Kurimoto M. (1997). Interleukin 18 induces the sequential activation of natural killer cells and cytotoxic T lymphocytes to protect syngeneic mice from transplantation with Meth A sarcoma. Cancer Res. ;57:4557-63.
Morinaga Y, Hayashi H, Takeuchi A, Onozaki K. (1990). Antiproliferative effect of interleukin 1 (IL-1) on tumor cells: G0-G1 arrest of a human melanoma cell line by IL-1. Biochem Biophys Res Commun. 173:186-92.
Nygren, P-A. (2008) Alternative binding proteins: affibody binding proteins developed from a small three-helix bundle scaffold. FEBS J. 275, 2668-2676.
Okamura H, Tsutsi H, Komatsu T, Yutsudo M, Hakura A, Tanimoto T, Torigoe K, Okura T, Nukada Y, Hattori K, et al. (1995). Cloning of a new cytokine that induces IFN-gamma production by T cells. Nature. 378:88-91.
Rangnekar VV, Waheed S, Rangnekar VM. (1992). Interleukin-1-inducible tumor growth arrest is characterized by activation of cell type-specific "early" gene expression programs. J Biol Chem. 267:6240-8.
Robertson MJ, Kirkwood JM, Logan TF, Koch KM, Kathman S, Kirby LC, Bell WN, Thurmond LM, Weisenbach J, Dar MM. (2008). A dose-escalation study of recombinant human interleukin-18 using two different schedules of administration in patients with cancer. Clin Cancer Res. 14:3462-9
Scatchard G. (1949). Ann New York Acad Sci 51, 660-72.
Schmitz J, Owyang A, Oldham E, Song Y, Murphy E, McClanahan TK, Zurawski G, Moshrefi M, Qin J, Li X, Gorman DM, Bazan JF, Kastelein RA. (2005). IL-33, an interleukin-1-like cytokine that signals via the IL-1 receptor-related protein ST2 and induces T helper type 2-associated cytokines. Immunity. 23:479-90.
Shaw MH, Kamada N, Kim YG, Núñez G. (2012). Microbiota-induced IL-1β, but not IL-6, is critical for the development of steady-state TH17 cells in the intestine. J Exp Med. 209:251-8.
Sims JE, Smith DE. The IL-1 family: regulators of immunity. (2010). Nat Rev Immunol. 10:89-102.
Skerra, A. (2008) Alternative binding proteins: anticalins - harnessing the structural plasticity of the lipocalin ligand pocket to engineer novel binding activities. FEBS J. 275, 2677-2683.
Stump, M.T., Binz, H.K., Amstutz, P. (2008) DARPins: a new generation of protein therapeutics. Drug iscov. Today 13, 695-701.
Sutton C, Brereton C, Keogh B, Mills KH, Lavelle EC. (2006). A crucial role for interleukin (IL)-1 in the induction of IL-17-producing T cells that mediate autoimmune encephalomyelitis. J Exp Med. 203:1685-91.
Takeda K, Tsutsui H, Yoshimoto T, Adachi O, Yoshida N, Kishimoto T, Okamura H, Nakanishi K, Akira S. (1998). Defective NK cell activity and Th1 response in IL-18-deficient mice. Immunity. 8:383-90.
Tramontano, A., Bianchi, E., Venturini, S., Martin, F., Pessi, A and Sollazzo, M. (1994) The making of the minibody: an engineered beta-protein for the display of conformationally constrained peptides. J. Mol. Recognition 7, 9-24.
Usui N, Mimnaugh EG, Sinha BK. (1991). A role for the interleukin 1 receptor in the synergistic antitumor effects of human interleukin 1 alpha and etoposide against human melanoma cells. Cancer Res. 1991 51:769-74.
Vanden Berghe W, Plaisance S, Boone E, De Bosscher K, Schmitz ML, Fiers W, Haegeman G. (1998). p38 and extracellular signal-regulated kinase mitogen-activated protein kinase pathways are required for nuclear factor-kappaB p65 transactivation mediated by tumor necrosis factor. J Biol Chem. 273:3285-90.
Vaneycken I, Devoogdt N, Van Gassen N, Vincke C, Xavier C, Wernery U, Muyldermans S, Lahoutte T, Caveliers V. (2011). Preclinical screening of anti-HER2 nanobodies for molecular imaging of breast cancer. FASEB J. 25:2433-46.
Wigginton JM, Lee JK, Wiltrout TA, Alvord WG, Hixon JA, Subleski J, Back TC, Wiltrout RH. (2002). Synergistic engagement of an ineffective endogenous anti-tumor immune response and induction of IFN-gamma and Fas-ligand-dependent tumor eradication by combined administration of IL-18 and IL-2. J Immunol. 169:4467-74.
Wilke CM, Bishop K, Fox D, Zou W. (2011). Deciphering the role of Th17 cells in human disease. Trends Immunol. 32:603-11.
Ye P, Rodriguez FH, Kanaly S, Stocking KL, Schurr J, Schwarzenberger P, Oliver P, Huang W, Zhang P, Zhang J, Shellito JE, Bagby GJ, Nelson S, Charrier K, Peschon JJ, Kolls JK. (2001). Requirement of interleukin 17 receptor signaling for lung CXC chemokine and granulocyte colony-stimulating factor expression, neutrophil recruitment, and host defense. J Exp Med. 194:519-27.
Zabeau L, Verhee A, Catteeuw D, Faes L, Seeuws S, Decruy T, Elewaut D, Peelman F, Tavernier J. (2012). Selection of non-competitive leptin antagonists using a random nanobody-based approach. Biochem J. 441:425-34.
Zaki MH, Vogel P, Body-Malapel M, Lamkanfi M, Kanneganti TD. (2010). IL-18 production downstream of the Nlrp3 inflammasome confers protection against colorectal tumor formation.

## Claims

1. A composition comprising a targeting construct, comprising:
(1) a mutated IL-1β **characterized by** a reduced affinity for its receptor as compared to wild type IL-1β, wherein the mutated IL-1β comprises one or more mutations selected from R120X, Q131G, L145G, H146X, Q148X, F162A, and K208E, where X is a non-conservative change, and
(2) a targeting moiety comprising a variable domain of camelid heavy chain antibodies (VHH) or a variable domain of new antigen receptors (VNAR).

2. The composition, according to claim 1, wherein said mutant IL-1β comprises one or more mutations selected from R120G, Q131G, L145G, H146A, Q148G, F162A, and K208E.

3. The composition, according to claim 1, wherein said targeting moiety is targeting to a marker expressed on an IL-1 R1 and/or IL-1 RacP expressing cell.

4. The composition according to any one of the preceding claims, wherein said targeting moiety is directed to a tissue specific marker.

5. The composition according to any one of the preceding claims, wherein said targeting moiety is directed to Her2 or leptin receptor.

6. The composition according to any of the preceding claims, wherein the mutated IL-1β comprises one or more of:
(i) Q131G and Q148G,
(ii) Q148G and K208E,
(iii) R120G and Q131G,
(iv) R120G and H146A,
(v) R120G and K208E,
(vi) R120G, F162A, and Q164E,
(vii) F162A and Q164E,
(viii) Q164E and E167K, and
(ix) L145A and L147A.

7. A composition according to any one of the preceding claims for use as a medicament.

8. The composition according to any one of the claims 1-6 for use in stimulation of the immune response.

9. The composition according to any one of the claims 1-6 for use in treatment of cancer.

10. A composition for use according to claim 8, wherein a cell is to be contacted with said composition.

11. The composition for use of claim 10, wherein
the mutated IL-1β comprises one or more of:
(i) Q131G and Q148G,
(ii) Q148G and K208E,
(iii) R120G and Q131G,
(iv) R120G and H146A,
(v) R120G and K208E,
(vi) R120G, F162A, and Q164E,
(vii) F162A and Q164E,
(viii) Q164E and E167K, and
(ix) L145A and L147A.

12. The composition for the use of claim 10 or 11, wherein the targeting moiety comprises a variable domain of a camelid heavy chain antibody (VHH) against HER2.

13. The composition for the use of claim 10 or 11, wherein the targeting moiety comprises a variable domain of a camelid heavy chain antibody (VHH) against leptin receptor.

## Patentansprüche

1. Eine Zusammensetzung umfassend ein Targeting-Konstrukt, umfassend:
(1) ein mutiertes IL-1β charakterisiert durch eine verminderte Affinität für seinen Rezeptor verglichen mit Wildtyp IL-1β, wobei das mutierte IL-1β eine oder mehrere Mutationen umfasst, ausgewählt aus R120X, Q131G, L145G, H146X, Q148X, F162A, und K208E, wobei X ein nicht-konservativer Austausch ist, und
(2) eine Targeting-Einheit, umfassend eine variable Domäne von schwerkettigen Kamel-Antikörpern (VHH) oder eine variable Domäne von neuen Antigen-Rezeptoren (VNAR).

2. Die Zusammensetzung nach Anspruch 1, wobei jenes mutierte IL-1β eine oder mehrere Mutationen umfasst, ausgewählt aus R120G, Q131G, L145G, H146A, Q148G, F162A, und K208E.

3. Die Zusammensetzung nach Anspruch1, wobei jene Targeting-Einheit einen Marker zum Ziel hat, der auf einer IL-1 R1 und/ oder IL-1 RacP exprimierenden Zelle exprimiert wird.

4. Die Zusammensetzung nach einem der vorherigen Ansprüche, wobei jene Targeting-Einheit gegen einen Gewebe-spezifischen Marker gerichtet ist.

5. Die Zusammensetzung nach einem der vorherigen Ansprüche, wobei jene Targeting-Einheit gegen HER2 oder den Leptin Rezeptor gerichtet ist.

6. Die Zusammensetzung nach einem der vorherigen Ansprüche, wobei das mutierte IL-1β einen oder mehrere von:
(i) Q131G und Q148G,
(ii) Q148G und K208E,
(iii) R120G und Q131G,
(iv) R120G und H146A,
(v) R120G und K208E,
(vi) R120G, F162A, und Q164E,
(vii) F162A und Q164E,
(viii) Q164E und E167K, und
(ix) L145A und L147A umfasst.

7. Die Zusammensetzung nach einem der vorherigen Ansprüche zur Verwendung als Medikament.

8. Die Zusammensetzung nach einem der Ansprüche 1-6 zur Verwendung bei der Stimulation einer Immunantwort.

9. Die Zusammensetzung nach einem der Ansprüche 1-6 zur Verwendung bei der Behandlung von Krebs.

10. Eine Zusammensetzung zur Verwendung nach Anspruch 8, wobei eine Zelle mit jener Komposition in Kontakt zu bringen ist.

11. Die Zusammensetzung zur Verwendung nach Anspruch 10, wobei das mutierte IL-1β eines oder mehrere von:
(i) Q131G und Q148G,
(ii) Q148G und K208E,
(iii) R120G und Q131G,
(iv) R120G und H146A,
(v) R120G und K208E,
(vi) R120G, F162A, und Q164E,
(vii) F162A und Q164E,
(viii) Q164E und E167K, und
(ix) L145A und L147A umfasst.

12. Die Zusammensetzung zur Verwendung nach Anspruch 10 oder 11, wobei die Targeting-Einheit eine variable Domäne eines schwerkettigen Kamelantikörpers (VHH) gerichtet gegen HER2 umfasst.

13. Die Zusammensetzung zur Verwendung nach Anspruch 10 oder 11, wobei die Targeting-Einheit eine variable Domäne eines schwerkettigen Kamel-Antikörpers (VHH) gerichtet gegen den Leptin Rezeptor umfasst.

## Revendications

1. Composition comprenant une construction de ciblage, comprenant :
(1) une IL-1β mutée **caractérisée par** une affinité réduite pour son récepteur par rapport à l'IL-1β de type sauvage, dans laquelle l'IL-1β mutée comprend une ou plusieurs mutations sélectionnées parmi R120X, Q131G, L145G, H146X, Q148X, F162A, et K208E, où X est un changement non conservatif, et
(2) un fragment de ciblage comprenant un domaine variable d'anticorps à chaîne lourde de camélidés (VHH) ou un domaine variable de nouveaux récepteurs d'antigène (VNAR).

2. Composition, selon la revendication 1, dans laquelle ladite IL-1β mutante comprend une ou plusieurs mutations sélectionnées parmi R120G, Q131G, L145G, H146A, Q148G, F162A, et K208E.

3. Composition, selon la revendication 1, dans laquelle le fragment de ciblage cible un marqueur exprimé sur une cellule exprimant IL-1 R1 et/ou IL-1 RacP.

4. Composition selon l'une quelconque des revendications précédentes, dans laquelle ledit fragment de ciblage est dirigé vers un marqueur spécifique d'un tissu.

5. Composition selon l'une quelconque des revendications précédentes, dans laquelle ledit fragment de ciblage est dirigé vers Her2 ou le récepteur de la leptine.

6. Composition selon l'une quelconque des revendications précédentes, dans laquelle l'IL-1β mutée comprend un ou plusieurs parmi :
(i) Q131G et Q148G,
(ii) Q148G et K208E,
(iii) R120G et Q131G,
(iv) R120G et H146A,
(v) R120G et K208E,
(vi) R120G, F162A, et Q164E,
(vii) F162A et Q164E,
(viii) Q164E et E167K, et
(ix) L145A et L147A.

7. Composition selon l'une quelconque des revendications précédentes pour son utilisation comme médicament.

8. Composition selon l'une quelconque des revendications 1 à 6 pour son utilisation dans la stimulation de la réponse immunitaire.

9. Composition selon l'une quelconque des revendications 1 à 6 pour son utilisation dans le traitement d'un cancer.

10. Composition pour son utilisation selon la revendication 8, dans laquelle une cellule doit être mise en contact avec ladite composition.

11. Composition pour son utilisation selon la revendication 10, dans laquelle
l'IL-1β mutée comprend un ou plusieurs parmi :
(i) Q131G et Q148G,
(ii) Q148G et K208E,
(iii) R120G et Q131G,
(iv) R120G et H146A,
(v) R120G et K208E,
(vi) R120G, F162A, et Q164E,
(vii) F162A et Q164E,
(viii) Q164E et E167K, et
(ix) L145A et L147A.

12. Composition pour l'utilisation selon la revendication 10 ou 11, dans laquelle le fragment de ciblage comprend un domaine variable d'un anticorps à chaîne lourde de camélidés (VHH) dirigé contre HER2.

13. Composition pour l'utilisation selon la revendication 10 ou 11, dans laquelle le fragment de ciblage comprend un domaine variable d'un anticorps à chaîne lourde de camélidés (VHH) dirigé contre le récepteur de la leptine.


### SEQUENCE LISTING

<110> VIB VZW UNIVERSITEIT GENT CENTRE NATIONAL DE LA RECHERCHE SCIENTIFIQUE UNIVERSITÉ MONTPELLIER 2 CENTRE HOSPITALIER REGIONAL UNIVERSITAIRE DE MONTPELLIER
<120> TARGETED MODIFIED IL-1 FAMILY MEMBERS
<130> JT/IL1/461
<150> EP 13306047.5
   <151> 2013-07-19
<160> 108
<170> PatentIn version 3.5
<210> 1
   <211> 44
   <212> DNA
   <213> Artificial Sequence
<220>
   <223> Primer
<400> 1
   ccgactacgc tggcggcagt gacggtgtca gaagcctgaa ctgc 44
<210> 2
   <211> 43
   <212> DNA
   <213> Artificial Sequence
<220>
   <223> Primer
<400> 2
   ctggcggcag cgcccctgtc gctagcctga actgcaccct gcg 43
<210> 3
   <211> 39
   <212> DNA
   <213> Artificial Sequence
<220>
   <223> Primer
<400> 3
   gcggcagcgc ccctgtcgga agcttgaact gcaccctgc 39
<210> 4
   <211> 52
   <212> DNA
   <213> Artificial Sequence
<220>
   <223> Primer
<400> 4
   cgctggcggc agtgcccctg tcagaagcgc gaactgcacc ctgcgggaca gc 52
<210> 5
   <211> 52
   <212> DNA
   <213> Artificial Sequence
<220>
   <223> Primer
<400> 5
   cgcccctgtc agaagcctga actgcggcgg ccgggacagc cagcagaaaa gc 52
<210> 6
   <211> 48
   <212> DNA
   <213> Artificial Sequence
<220>
   <223> Primer
<400> 6
   agaagcctga actgcacact gggggacagc cagcagaaaa gcctggtc 48
<210> 7
   <211> 30
   <212> DNA
   <213> Artificial Sequence
<220>
   <223> Primer
<400> 7
   ccctgcggga cagcgcgcag aaaagcctgg 30
<210> 8
   <211> 43
   <212> DNA
   <213> Artificial Sequence
<220>
   <223> Primer
<400> 8
   ctgcaccctg cgggacagct ggcagaaaag cctggtcatg agc 43
<210> 9
   <211> 37
   <212> DNA
   <213> Artificial Sequence
<220>
   <223> Primer
<400> 9
   ctgcgggaca gccaggggaa gagcctggtc atgagcg 37
<210> 10
   <211> 45
   <212> DNA
   <213> Artificial Sequence
<220>
   <223> Primer
<400> 10
   gcaccctgcg ggacagccag caggctagcc tggtcatgag cggcc 45
<210> 11
   <211> 49
   <212> DNA
   <213> Artificial Sequence
<220>
   <223> Primer
<400> 11
   cagcagaaaa gcctggtcat ggggtacccc tacgagctga aggcactgc 49
<210> 12
   <211> 45
   <212> DNA
   <213> Artificial Sequence
<220>
   <223> Primer
<400> 12
   gcccctacga gctgaaggca ggtcatctgc agggccagga catgg 45
<210> 13
   <211> 38
   <212> DNA
   <213> Artificial Sequence
<220>
   <223> Primer
<400> 13
   cgagctgaag gcactggctc ttcagggcca ggacatgg 38
<210> 14
   <211> 42
   <212> DNA
   <213> Artificial Sequence
<220>
   <223> Primer
<400> 14
   cctacgagct gaaggcactg ggtctgcagg gccaggacat gg 42
<210> 15
   <211> 29
   <212> DNA
   <213> Artificial Sequence
<220>
   <223> Primer
<400> 15
   gctgaaggca ctggagctgc agggccagg 29
<210> 16
   <211> 29
   <212> DNA
   <213> Artificial Sequence
<220>
   <223> Primer
<400> 16
   agctgaaggc actgaatctg cagggccag 29
<210> 17
   <211> 27
   <212> DNA
   <213> Artificial Sequence
<220>
   <223> Primer
<400> 17
   ctgaaggcac tgcgtctgca gggccag 27
<210> 18
   <211> 48
   <212> DNA
   <213> Artificial Sequence
<220>
   <223> Primer
<400> 18
   gcggccccta cgagctgaag gcagcgcatg cgcagggcca ggacatgg 48
<210> 19
   <211> 27
   <212> DNA
   <213> Artificial Sequence
<220>
   <223> Primer
<400> 19
   ggcactgcat ctggagggcc aggacat 27
<210> 20
   <211> 38
   <212> DNA
   <213> Artificial Sequence
<220>
   <223> Primer
<400> 20
   gaaggcactg catctgggtg gccaggacat ggaacagc 38
<210> 21
   <211> 27
   <212> DNA
   <213> Artificial Sequence
<220>
   <223> Primer
<400> 21
   gcactgcatc tgctgggcca ggacatg 27
<210> 22
   <211> 47
   <212> DNA
   <213> Artificial Sequence
<220>
   <223> Primer
<400> 22
   cgagctgaag gcactgcatc tggggggcgg ggacatggaa cagcagg 47
<210> 23
   <211> 48
   <212> DNA
   <213> Artificial Sequence
<220>
   <223> Primer
<400> 23
   gcactgcatc tgcagggcgg ggccatggaa cagcaggtcg tgttcagc 48
<210> 24
   <211> 54
   <212> DNA
   <213> Artificial Sequence
<220>
   <223> Primer
<400> 24
   gcactgcatc tgcagggcca ggacggggaa cagcaggtgg tgttcagcat gagc 54
<210> 25
   <211> 58
   <212> DNA
   <213> Artificial Sequence
<220>
   <223> Primer
<400> 25
   catggaacag caggtggtgt tcagcatgag cgccgtgcag ggcgaggaaa gcaacgac 58
<210> 26
   <211> 56
   <212> DNA
   <213> Artificial Sequence
<220>
   <223> Primer
<400> 26
   gcaggtcgtg ttcagcatga gcgccgtgga gggcgaggaa agcaatgaca agatcc 56
<210> 27
   <211> 46
   <212> DNA
   <213> Artificial Sequence
<220>
   <223> Primer
<400> 27
   ccgacttcac catgcaggcc gtctccagcg gcggcagcag atctgg 46
<210> 28
   <211> 50
   <212> DNA
   <213> Artificial Sequence
<220>
   <223> Primer
<400> 28
   gcatgagctt cgtggggggc aaggaaagca atgacaagat ccccgtggcc 50
<210> 29
   <211> 55
   <212> DNA
   <213> Artificial Sequence
<220>
   <223> Primer
<400> 29
   gcagggcgag gaaagcggcg gcaagatccc cgtggcccta ggcctgaaag agaag 55
<210> 30
   <211> 32
   <212> DNA
   <213> Artificial Sequence
<220>
   <223> Primer
<400> 30
   gaaagcaacg acaaggcccc cgtggccctg gg 32
<210> 31
   <211> 36
   <212> DNA
   <213> Artificial Sequence
<220>
   <223> Primer
<400> 31
   gcaacgacaa gatccccgcg gccctgggcc tgaaag 36
<210> 32
   <211> 55
   <212> DNA
   <213> Artificial Sequence
<220>
   <223> Primer
<400> 32
   gcagctggaa agcgtggatc ccaagaacta ccccgagaaa aagatggaaa aacgc 55
<210> 33
   <211> 49
   <212> DNA
   <213> Artificial Sequence
<220>
   <223> Primer
<400> 33
   ccccaagaac taccccaagg caaagatgga aaagcgcttc gtgttcaac 49
<210> 34
   <211> 55
   <212> DNA
   <213> Artificial Sequence
<220>
   <223> Primer
<400> 34
   gcagctggaa agcgtggatc ccaagaacta ccccaaggac aagatggaaa aacgc 55
<210> 35
   <211> 46
   <212> DNA
   <213> Artificial Sequence
<220>
   <223> Primer
<400> 35
   ccccaagaac taccccaagg cagcgatgga aaaacgcttc gtgttc 46
<210> 36
   <211> 44
   <212> DNA
   <213> Artificial Sequence
<220>
   <223> Primer
<400> 36
   aaaaacgctt cgtgttcaac agcatcgaga tcaacaacaa gctc 44
<210> 37
   <211> 41
   <212> DNA
   <213> Artificial Sequence
<220>
   <223> Primer
<400> 37
   aaaaacgctt cgtgttcaac cagatcgaga tcaacaacaa g 41
<210> 38
   <211> 42
   <212> DNA
   <213> Artificial Sequence
<220>
   <223> Primer
<400> 38
   gcttcgtgtt caacaagatc tcgatcaaca acaagctcga gt 42
<210> 39
   <211> 39
   <212> DNA
   <213> Artificial Sequence
<220>
   <223> Primer
<400> 39
   cttcgtgttc aacaagatca agatcaacaa caagctcga 39
<210> 40
   <211> 53
   <212> DNA
   <213> Artificial Sequence
<220>
   <223> Primer
<400> 40
   ggaaaaacgc ttcgtcttca acagcatctc gatcaacaac aagctcgagt tcg 53
<210> 41
   <211> 48
   <212> DNA
   <213> Artificial Sequence
<220>
   <223> Primer
<400> 41
   cgcttcgtgt tcaacaagat ctcgatcaac gccaagctcg agttcgag 48
<210> 42
   <211> 46
   <212> DNA
   <213> Artificial Sequence
<220>
   <223> Primer
<400> 42
   caacaagatc gagatcaaca gcagcctcga attcgagagc gcccag 46
<210> 43
   <211> 50
   <212> DNA
   <213> Artificial Sequence
<220>
   <223> Primer
<400> 43
   ccccaactgg tacatcagta ctagtcaggc caagaatatg cccgtgttcc 50
<210> 44
   <211> 46
   <212> DNA
   <213> Artificial Sequence
<220>
   <223> Primer
<400> 44
   cagcactagt caggccgagc agatgcccgt cttcctgggc ggcacc 46
<210> 45
   <211> 49
   <212> DNA
   <213> Artificial Sequence
<220>
   <223> Primer
<400> 45
   catcagcact agtcaggcca agcagatgcc cgtcttcctg ggcggcacc 49
<210> 46
   <211> 39
   <212> DNA
   <213> Artificial Sequence
<220>
   <223> Primer
<400> 46
   gcggcagcgc ccctgtcgga agcttgaact gcaccctgc 39
<210> 47
   <211> 38
   <212> DNA
   <213> Artificial Sequence
<220>
   <223> Primer
<400> 47
   cgagctgaag gcactggctc ttcagggcca ggacatgg 38
<210> 48
   <211> 48
   <212> DNA
   <213> Artificial Sequence
<220>
   <223> Primer
<400> 48
   gcggccccta cgagctgaag gcagcgcatg cgcagggcca ggacatgg 48
<210> 49
   <211> 39
   <212> DNA
   <213> Artificial Sequence
<220>
   <223> Primer
<400> 49
   gcggcagcgc ccctgtcgga agcttgaact gcaccctgc 39
<210> 50
   <211> 56
   <212> DNA
   <213> Artificial Sequence
<220>
   <223> Primer
<400> 50
   gcaggtcgtg ttcagcatga gcgccgtgga gggcgaggaa agcaatgaca agatcc 56
<210> 51
   <211> 55
   <212> DNA
   <213> Artificial Sequence
<220>
   <223> Primer
<400> 51
   gcagctggaa agcgtggatc ccaagaacta ccccgagaaa aagatggaaa aacgc 55
<210> 52
   <211> 37
   <212> DNA
   <213> Artificial Sequence
<220>
   <223> Primer
<400> 52
   ctgcgggaca gccaggggaa gagcctggtc atgagcg 37
<210> 53
   <211> 56
   <212> DNA
   <213> Artificial Sequence
<220>
   <223> Primer
<400> 53
   gcaggtcgtg ttcagcatga gcgccgtgga gggcgaggaa agcaatgaca agatcc 56
<210> 54
   <211> 55
   <212> DNA
   <213> Artificial Sequence
<220>
   <223> Primer
<400> 54
   gcagctggaa agcgtggatc ccaagaacta ccccgagaaa aagatggaaa aacgc 55
<210> 55
   <211> 44
   <212> DNA
   <213> Artificial Sequence
<220>
   <223> Primer
<400> 55
   gcagttcagg cttctgacac cgtcactgcc gccagcgtag tcgg 44
<210> 56
   <211> 43
   <212> DNA
   <213> Artificial Sequence
<220>
   <223> Primer
<400> 56
   cgcagggtgc agttcaggct agcgacaggg gcgctgccgc cag 43
<210> 57
   <211> 39
   <212> DNA
   <213> Artificial Sequence
<220>
   <223> Primer
<400> 57
   gcagggtgca gttcaagctt ccgacagggg cgctgccgc 39
<210> 58
   <211> 52
   <212> DNA
   <213> Artificial Sequence
<220>
   <223> Primer
<400> 58
   gctgtcccgc agggtgcagt tcgcgcttct gacaggggca ctgccgccag cg 52
<210> 59
   <211> 52
   <212> DNA
   <213> Artificial Sequence
<220>
   <223> Primer
<400> 59
   gcttttctgc tggctgtccc ggccgccgca gttcaggctt ctgacagggg cg 52
<210> 60
   <211> 48
   <212> DNA
   <213> Artificial Sequence
<220>
   <223> Primer
<400> 60
   gaccaggctt ttctgctggc tgtcccccag tgtgcagttc aggcttct 48
<210> 61
   <211> 30
   <212> DNA
   <213> Artificial Sequence
<220>
   <223> Primer
<400> 61
   ccaggctttt ctgcgcgctg tcccgcaggg 30
<210> 62
   <211> 43
   <212> DNA
   <213> Artificial Sequence
<220>
   <223> Primer
<400> 62
   gctcatgacc aggcttttct gccagctgtc ccgcagggtg cag 43
<210> 63
   <211> 37
   <212> DNA
   <213> Artificial Sequence
<220>
   <223> Primer
<400> 63
   cgctcatgac caggctcttc ccctggctgt cccgcag 37
<210> 64
   <211> 45
   <212> DNA
   <213> Artificial Sequence
<220>
   <223> Primer
<400> 64
   ggccgctcat gaccaggcta gcctgctggc tgtcccgcag ggtgc 45
<210> 65
   <211> 49
   <212> DNA
   <213> Artificial Sequence
<220>
   <223> Primer
<400> 65
   gcagtgcctt cagctcgtag gggtacccca tgaccaggct tttctgctg 49
<210> 66
   <211> 45
   <212> DNA
   <213> Artificial Sequence
<220>
   <223> Primer
<400> 66
   ccatgtcctg gccctgcaga tgacctgcct tcagctcgta ggggc 45
<210> 67
   <211> 38
   <212> DNA
   <213> Artificial Sequence
<220>
   <223> Primer
<400> 67
   ccatgtcctg gccctgaaga gccagtgcct tcagctcg 38
<210> 68
   <211> 42
   <212> DNA
   <213> Artificial Sequence
<220>
   <223> Primer
<400> 68
   ccatgtcctg gccctgcaga cccagtgcct tcagctcgta gg 42
<210> 69
   <211> 29
   <212> DNA
   <213> Artificial Sequence
<220>
   <223> Primer
<400> 69
   cctggccctg cagctccagt gccttcagc 29
<210> 70
   <211> 29
   <212> DNA
   <213> Artificial Sequence
<220>
   <223> Primer
<400> 70
   ctggccctgc agattcagtg ccttcagct 29
<210> 71
   <211> 27
   <212> DNA
   <213> Artificial Sequence
<220>
   <223> Primer
<400> 71
   ctggccctgc agacgcagtg ccttcag 27
<210> 72
   <211> 48
   <212> DNA
   <213> Artificial Sequence
<220>
   <223> Primer
<400> 72
   ccatgtcctg gccctgcgca tgcgctgcct tcagctcgta ggggccgc 48
<210> 73
   <211> 27
   <212> DNA
   <213> Artificial Sequence
<220>
   <223> Primer
<400> 73
   atgtcctggc cctccagatg cagtgcc 27
<210> 74
   <211> 38
   <212> DNA
   <213> Artificial Sequence
<220>
   <223> Primer
<400> 74
   gctgttccat gtcctggcca cccagatgca gtgccttc 38
<210> 75
   <211> 27
   <212> DNA
   <213> Artificial Sequence
<220>
   <223> Primer
<400> 75
   catgtcctgg cccagcagat gcagtgc 27
<210> 76
   <211> 47
   <212> DNA
   <213> Artificial Sequence
<220>
   <223> Primer
<400> 76
   cctgctgttc catgtccccg ccccccagat gcagtgcctt cagctcg 47
<210> 77
   <211> 48
   <212> DNA
   <213> Artificial Sequence
<220>
   <223> Primer
<400> 77
   gctgaacacg acctgctgtt ccatggcccc gccctgcaga tgcagtgc 48
<210> 78
   <211> 54
   <212> DNA
   <213> Artificial Sequence
<220>
   <223> Primer
<400> 78
   gctcatgctg aacaccacct gctgttcccc gtcctggccc tgcagatgca gtgc 54
<210> 79
   <211> 58
   <212> DNA
   <213> Artificial Sequence
<220>
   <223> Primer
<400> 79
   gtcgttgctt tcctcgccct gcacggcgct catgctgaac accacctgct gttccatg 58
<210> 80
   <211> 56
   <212> DNA
   <213> Artificial Sequence
<220>
   <223> Primer
<400> 80
   ggatcttgtc attgctttcc tcgccctcca cggcgctcat gctgaacacg acctgc 56
<210> 81
   <211> 46
   <212> DNA
   <213> Artificial Sequence
<220>
   <223> Primer
<400> 81
   ccagatctgc tgccgccgct ggagacggcc tgcatggtga agtcgg 46
<210> 82
   <211> 50
   <212> DNA
   <213> Artificial Sequence
<220>
   <223> Primer
<400> 82
   ggccacgggg atcttgtcat tgctttcctt gccccccacg aagctcatgc 50
<210> 83
   <211> 55
   <212> DNA
   <213> Artificial Sequence
<220>
   <223> Primer
<400> 83
   cttctctttc aggcctaggg ccacggggat cttgccgccg ctttcctcgc cctgc 55
<210> 84
   <211> 32
   <212> DNA
   <213> Artificial Sequence
<220>
   <223> Primer
<400> 84
   cccagggcca cgggggcctt gtcgttgctt tc 32
<210> 85
   <211> 36
   <212> DNA
   <213> Artificial Sequence
<220>
   <223> Primer
<400> 85
   ctttcaggcc cagggccgcg gggatcttgt cgttgc 36
<210> 86
   <211> 55
   <212> DNA
   <213> Artificial Sequence
<220>
   <223> Primer
<400> 86
   gcgtttttcc atctttttct cggggtagtt cttgggatcc acgctttcca gctgc 55
<210> 87
   <211> 49
   <212> DNA
   <213> Artificial Sequence
<220>
   <223> Primer
<400> 87
   gttgaacacg aagcgctttt ccatctttgc cttggggtag ttcttgggg 49
<210> 88
   <211> 55
   <212> DNA
   <213> Artificial Sequence
<220>
   <223> Primer
<400> 88
   gcgtttttcc atcttgtcct tggggtagtt cttgggatcc acgctttcca gctgc 55
<210> 89
   <211> 46
   <212> DNA
   <213> Artificial Sequence
<220>
   <223> Primer
<400> 89
   gaacacgaag cgtttttcca tcgctgcctt ggggtagttc ttgggg 46
<210> 90
   <211> 44
   <212> DNA
   <213> Artificial Sequence
<220>
   <223> Primer
<400> 90
   gagcttgttg ttgatctcga tgctgttgaa cacgaagcgt tttt 44
<210> 91
   <211> 41
   <212> DNA
   <213> Artificial Sequence
<220>
   <223> Primer
<400> 91
   cttgttgttg atctcgatct ggttgaacac gaagcgtttt t 41
<210> 92
   <211> 42
   <212> DNA
   <213> Artificial Sequence
<220>
   <223> Primer
<400> 92
   actcgagctt gttgttgatc gagatcttgt tgaacacgaa gc 42
<210> 93
   <211> 39
   <212> DNA
   <213> Artificial Sequence
<220>
   <223> Primer
<400> 93
   tcgagcttgt tgttgatctt gatcttgttg aacacgaag 39
<210> 94
   <211> 53
   <212> DNA
   <213> Artificial Sequence
<220>
   <223> Primer
<400> 94
   cgaactcgag cttgttgttg atcgagatgc tgttgaagac gaagcgtttt tcc 53
<210> 95
   <211> 48
   <212> DNA
   <213> Artificial Sequence
<220>
   <223> Primer
<400> 95
   ctcgaactcg agcttggcgt tgatcgagat cttgttgaac acgaagcg 48
<210> 96
   <211> 46
   <212> DNA
   <213> Artificial Sequence
<220>
   <223> Primer
<400> 96
   ctgggcgctc tcgaattcga ggctgctgtt gatctcgatc ttgttg 46
<210> 97
   <211> 50
   <212> DNA
   <213> Artificial Sequence
<220>
   <223> Primer
<400> 97
   ggaacacggg catattcttg gcctgactag tactgatgta ccagttgggg 50
<210> 98
   <211> 46
   <212> DNA
   <213> Artificial Sequence
<220>
   <223> Primer
<400> 98
   ggtgccgccc aggaagacgg gcatctgctc ggcctgacta gtgctg 46
<210> 99
   <211> 49
   <212> DNA
   <213> Artificial Sequence
<220>
   <223> Primer
<400> 99
   ggtgccgccc aggaagacgg gcatctgctt ggcctgacta gtgctgatg 49
<210> 100
   <211> 39
   <212> DNA
   <213> Artificial Sequence
<220>
   <223> Primer
<400> 100
   gcagggtgca gttcaagctt ccgacagggg cgctgccgc 39
<210> 101
   <211> 38
   <212> DNA
   <213> Artificial Sequence
<220>
   <223> Primer
<400> 101
   ccatgtcctg gccctgaaga gccagtgcct tcagctcg 38
<210> 102
   <211> 48
   <212> DNA
   <213> Artificial Sequence
<220>
   <223> Primer
<400> 102
   ccatgtcctg gccctgcgca tgcgctgcct tcagctcgta ggggccgc 48
<210> 103
   <211> 39
   <212> DNA
   <213> Artificial Sequence
<220>
   <223> Primer
<400> 103
   gcagggtgca gttcaagctt ccgacagggg cgctgccgc 39
<210> 104
   <211> 56
   <212> DNA
   <213> Artificial Sequence
<220>
   <223> Primer
<400> 104
   ggatcttgtc attgctttcc tcgccctcca cggcgctcat gctgaacacg acctgc 56
<210> 105
   <211> 55
   <212> DNA
   <213> Artificial Sequence
<220>
   <223> Primer
<400> 105
   gcgtttttcc atctttttct cggggtagtt cttgggatcc acgctttcca gctgc 55
<210> 106
   <211> 37
   <212> DNA
   <213> Artificial Sequence
<220>
   <223> Primer
<400> 106
   cgctcatgac caggctcttc ccctggctgt cccgcag 37
<210> 107
   <211> 56
   <212> DNA
   <213> Artificial Sequence
<220>
   <223> Primer
<400> 107
   ggatcttgtc attgctttcc tcgccctcca cggcgctcat gctgaacacg acctgc 56
<210> 108
   <211> 55
   <212> DNA
   <213> Artificial Sequence
<220>
   <223> Primer
<400> 108
   gcgtttttcc atctttttct cggggtagtt cttgggatcc acgctttcca gctgc 55